# EUROPEAN PATENT APPLICATION

(11) **EP 4 806 909 A1**
(43) Date of publication of application: **16.09.2026**
(21) Application number: 24884780.8
(22) Date of filing: 30.10.2024
(51) Int. Cl.: C07K 14/605, A61K 47/54, A61P 3/10, A61K 38/26

(54) **GLP-1 COMPOUND, AND PREPARATION METHOD THEREFOR AND USE THEREOF**

(30) Priority: 31.10.2023 CN 202311441329; 16.11.2023 CN 202311537271; 17.11.2023 CN 202311536917; 15.12.2023 CN 202311738354; 15.12.2023 CN 202311736180; 02.02.2024 CN 202410162644; 02.02.2024 CN 202410165153; 02.02.2024 CN 202410162317; 20.03.2024 CN 202410338571; 20.03.2024 CN 202410327708; 28.05.2024 CN 202410678200; 28.05.2024 CN 202410678866; 28.05.2024 CN 202410681438
(71) Applicant: Shenzhen Salubris Pharmaceuticals Co., Ltd., Shenzhen, Guangdong 518017 (CN)
(72) Inventor: XU, Wenjie, Shenzhen, Guangdong 518017 (CN); LIAN, Xiaolei, Shenzhen, Guangdong 518017 (CN); XING, Wei, Shenzhen, Guangdong 518017 (CN); ZHANG, Shu, Shenzhen, Guangdong 518017 (CN); XIAO, Ying, Shenzhen, Guangdong 518017 (CN); CAO, Dapeng, Shenzhen, Guangdong 518017 (CN); ZHU, Ruihua, Shenzhen, Guangdong 518017 (CN); HUA, Huaijie, Shenzhen, Guangdong 518017 (CN); LI, Qinze, Shenzhen, Guangdong 518017 (CN)
(74) Representative: karo IP
(86) International application number: PCT/CN2024/128351
(87) International publication number: WO 2025/092778

(57) **Abstract**

The present invention belongs to the technical field of therapeutic peptides, and relates to a compound as shown in general formula (1), or a racemate thereof, or an isomer thereof, or a pharmaceutically acceptable salt thereof, as a GLP-1 compound, and the use thereof in the treatment of diseases.

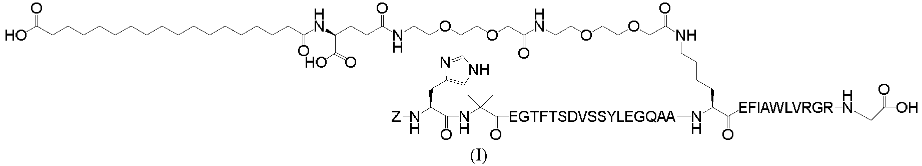

## Description

### TECHNICAL FIELD

The present invention belongs to the technical field of therapeutic peptides, and specifically relates to GLP-1 compounds, and preparation method thereof and use thereof.

### BACKGROUND ART

One of the main challenges for oral delivery of proteins and peptides is that these compounds cannot readily cross gastrointestinal membranes. Human GLP-1 and its analogs have low oral bioavailability. After oral administration, the exposure and bioavailability of human GLP-1 and its analogs are extremely low. Human GLP-1 and its analogs can only achieve therapeutically relevant plasma concentrations after oral administration when formulated with specific amounts of certain absorption enhancer(s).

Currently available oral GLP-1 receptor agonist drugs must be administered once daily. Such relatively high dosing frequency is not conducive to improved patient convenience and improved patient compliance. Therefore, developing an oral GLP-1 receptor agonist suitable for administration at a frequency lower than once daily would represent a significant improvement to available treatment options.

### SUMMARY OF THE INVENTION

In view of the problems existing in the prior art, the present invention provides a GLP-1 compound, or an isomer thereof, or a racemate thereof, or a pharmaceutically acceptable salt thereof, and a preparation method thereof and use thereof, which has improved activity, bioavailability, efficacy, etc.

In a first aspect, the present invention provides a GLP-1 compound shown in formula (I), or an isomer thereof, or a racemate thereof, or a pharmaceutically acceptable salt thereof, comprising: 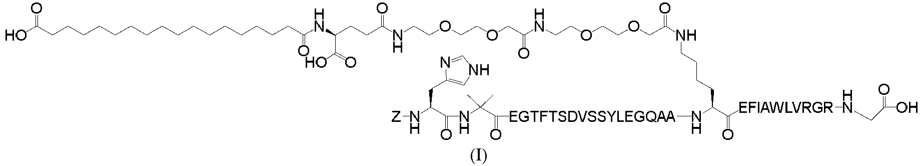
wherein, Z is selected from 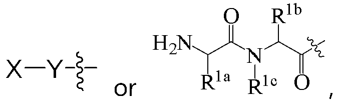 X and Y are each independently selected from 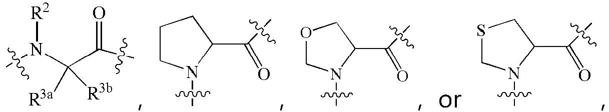 one of said X and Y is selected from 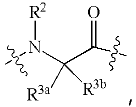 and a bond in the terminal group of X is linked to a hydrogen;
said R^{1a}, R^{1b}, R^{1c}, R², R^{3a}, R^{3b} are each independently selected from hydrogen, C₁₋₆ alkyl, C₃₋₆ cycloalkyl, or 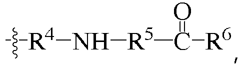 or alternatively, R^{3a} and R^{3b} are cyclized, together with the atom to which they are attached, form a C₃₋₆ cycloalkyl; at least one of R^{1a}, R^{1b}, R^{1c}, R², R^{3a} and R^{3b} is selected from 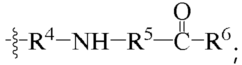
said R⁴ is selected from C₁₋₆ alkyl, or 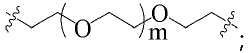
R⁵ is selected from one of 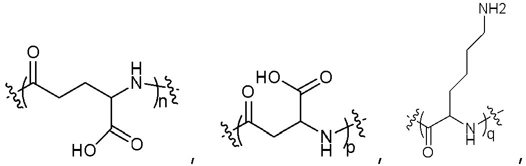 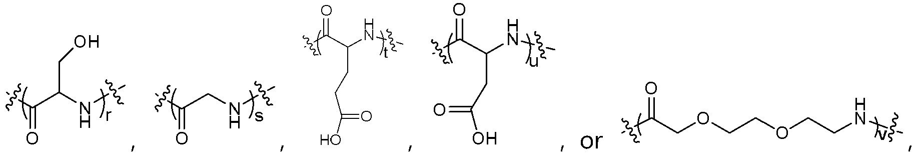 or any combination of two or more thereof;
wherein, when R^{1a} and R^{1b} are selected from hydrogen, R^{1c} is selected from 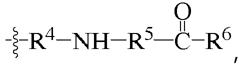 then R⁴ is selected from 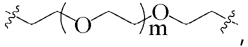 or R⁵ is selected from 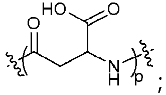
R⁶ is selected from a C₁₀₋₂₀ fatty acid;
m and n are integers, preferably integers from 0-10; p, q, r, s, t, u, v are selected from 1, 2, 3 or 4.

In a second aspect, the present invention further provides a pharmaceutical composition comprising a therapeutically effective amount of the compound according to any one of the above, or a pharmaceutically acceptable salt thereof, and a pharmaceutically acceptable carrier.

In a third aspect, the present invention further provides use of a therapeutically effective amount of the aforementioned compound or a pharmaceutically acceptable salt thereof in the manufacture of a medicament for treating a disease, said disease being a chronic related disease selected from diabetes, obesity, non-alcoholic fatty liver disease and non-alcoholic steatohepatitis, cardiovascular disease, neurodegenerative disorders, chronic kidney disease, diabetic nephropathy, peripheral arterial disease, and/or heart failure.

Specifically, the present invention is achieved through the following technical solutions:
A GLP-1 compound shown in formula (I), or an isomer thereof, or a racemate thereof, or a pharmaceutically acceptable salt thereof, comprising: 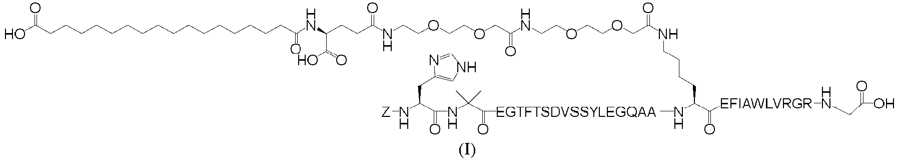
wherein, Z is selected from 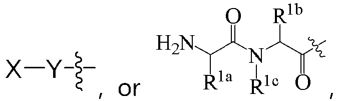 X and Y are each independently selected from 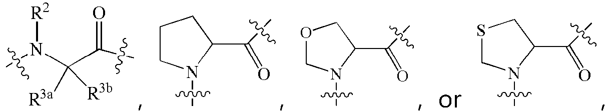 one of said X and Y is selected from 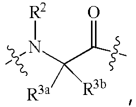 and a bond in the terminal group of X is linked to a hydrogen;
said R^{1a}, R^{1b}, R^{1c}, R², R^{3a}, R^{3b} are each independently selected from hydrogen, C₁₋₆ alkyl, C₃₋₆ cycloalkyl, or 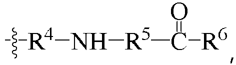 or alternatively, said R^{3a} and R^{3b} are cyclized, together with the atom to which they are attached, form a C₃₋₆ cycloalkyl; at least one of R^{1a}, R^{1b}, R^{1c}, R², R^{3a} and R^{3b} is selected from 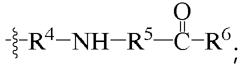
said R⁴ is selected from C₁₋₆ alkyl, or 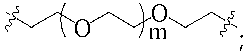
R⁵ is selected from one of 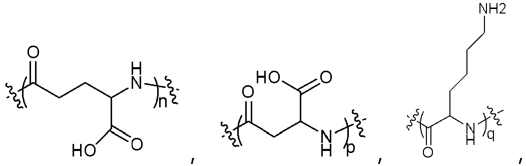 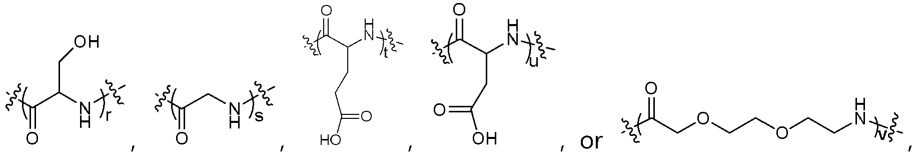 or any combination of two or more thereof;
wherein, when R^{1a} and R^{1b} are selected from hydrogen, R^{1c} is selected from 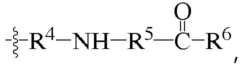 then R⁴ is selected from 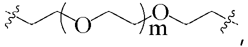 or R⁵ is selected from 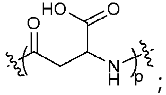
R⁶ is selected from a C₁₀₋₂₀ fatty acid;
m and n are integers, preferably integers from 0-10; p, q, r, s, t, u, v are selected from 1, 2, 3 or 4;
wherein, when a bond in the terminal group of X is linked to a hydrogen, X is selected from

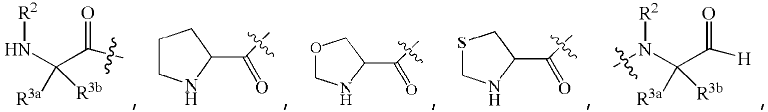

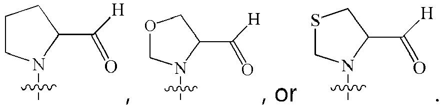

As a preferable technical solution of the present invention, when Z is selected from 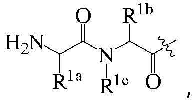 R^{1c} is selected from 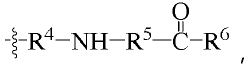 the GLP-1 compound is selected from a compound shown in formula (II): 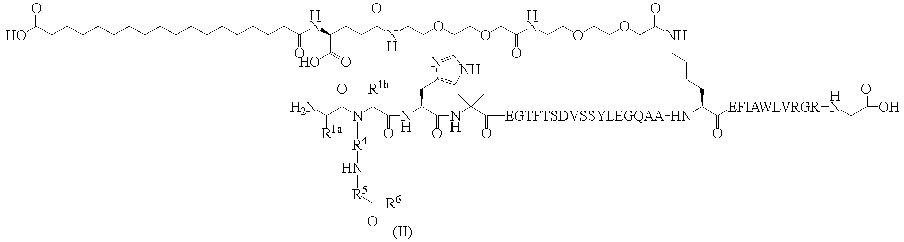
wherein, R^{1a} and R^{1b} are selected from hydrogen, or C₁₋₆ alkyl;
R⁴ is selected from C₁₋₆ alkyl, or 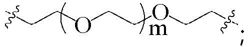
R⁵ is selected from 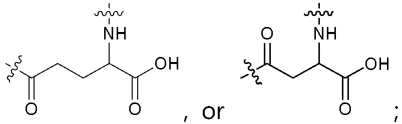
when R^{1a} and R^{1b} are selected from hydrogen, then R⁴ is selected from 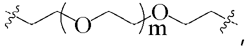 or R⁵ is selected from 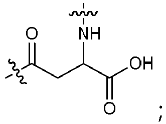
R⁶ is selected from a C₁₀₋₂₀ fatty acid;
m is selected from 0, 1, 2, 3 or 4.

As a preferable technical solution of the present invention, when Z is selected from 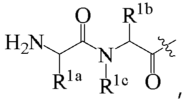 the GLP-1 compound is selected from a compound shown in formula (III): 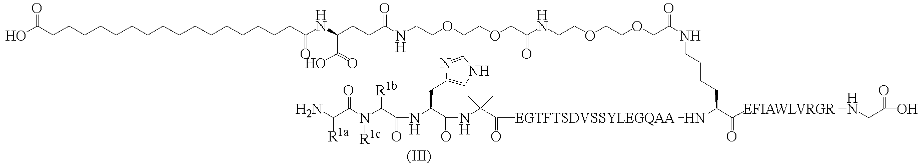
wherein, R^{1a} and R^{1b} are independently selected from hydrogen, C₁₋₆ alkyl, or 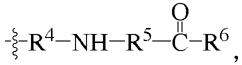 and said R^{1a} and R^{1b} are not simultaneously hydrogen or C₁₋₆ alkyl;
R^{1c} is selected from hydrogen, or C₁₋₆ alkyl;
R⁴ is selected from C₁₋₆ alkyl, or 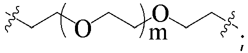
R⁵ is selected from one of 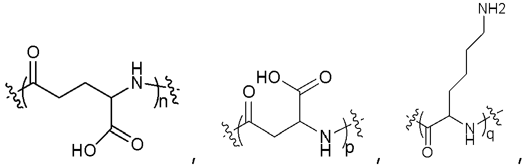 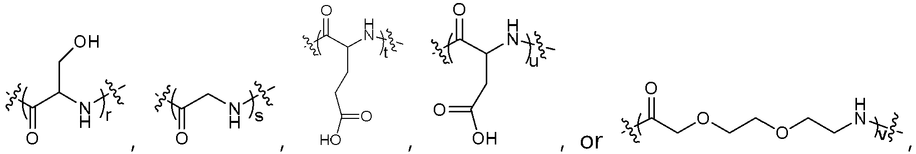 or any combination of two or more thereof;
R⁶ is selected from a C₁₀₋₂₀ fatty acid;
m is selected from 0, 1, 2, 3, or 4; n, p, q, r, s, t, u, v are selected from 1, 2, 3 or 4.

As a preferable technical solution of the present invention, when Z is selected from 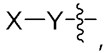 the GLP-1 compound is selected from a compound shown in formula (VI): 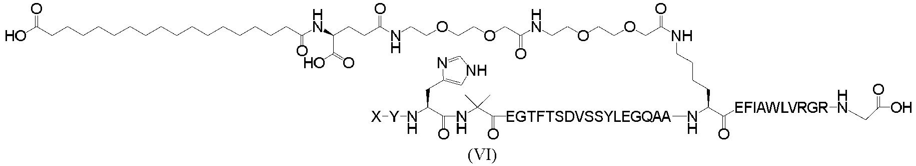
wherein, X and Y are independently selected from 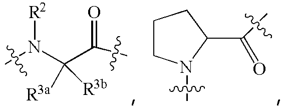 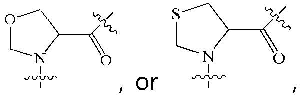 one of said X and Y is selected from 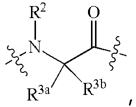 and a bond in the terminal group of X is linked to a hydrogen;
said R² is selected from hydrogen, C₁₋₆ alkyl, C₃₋₆ cycloalkyl, or 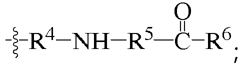
said R^{3a} and R^{3b} are each independently selected from hydrogen, C₁₋₆ alkyl, or 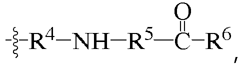 or alternatively, R^{3a} and R^{3b} are cyclized, together with the atom to which they are attached, form a C₃₋₆ cycloalkyl, at least one of said R², R^{3a} and R^{3b} is selected from 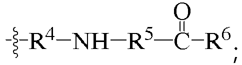
said R⁴ is selected from C₁₋₆ alkyl, or 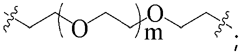
said R⁵ is selected from one of 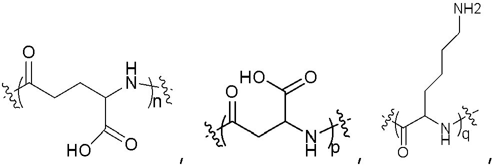 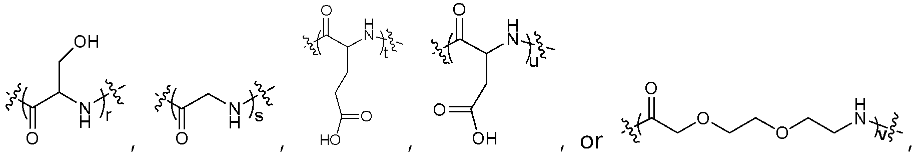 or any combination of two or more thereof;
R⁶ is selected from a C₁₀₋₂₀ fatty acid;
m is selected from 0, 1, 2, 3, or 4; n, p, q, r, s, t, u, v are selected from 1, 2, 3 or 4.

As a preferable technical solution of the present invention, said C₁₋₆ alkyl is selected from methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, iso-pentyl, neopentyl, n-hexyl, iso-hexyl, sec-hexyl, tert-hexyl, neo-hexyl, 2-methylpentyl, 1,2-dimethylbutyl, and 1-ethylbutyl.

As a preferable technical solution of the present invention, said C₃₋₆ cycloalkyl is selected from cyclopropyl, cyclobutyl, cyclopentyl, and cyclohexyl.

As a preferable technical solution of the present invention, wherein, m is 0 or 1, n is 1, p is 1;
said R^{1a}, R^{1b}, R^{1c}, R², R^{3a}, R^{3b} are each independently selected from hydrogen, methyl, or 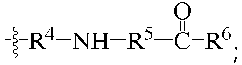
said R⁴ is selected from ethyl, butyl, or 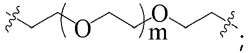
said R⁵ is selected from 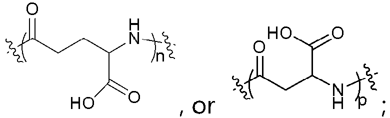
when R^{1a} and R^{1b} are selected from hydrogen, R^{1c} is selected from 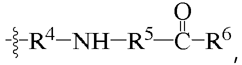 then R⁴ is selected from 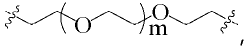 or R⁵ is selected from

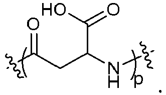

As a preferable technical solution of the present invention, m is 0 or 1;
when said R^{1a} and R^{1b} are selected from hydrogen, then said R⁴ is selected from 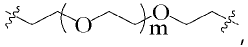 or R⁵ is selected from 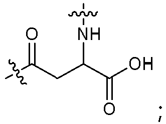
when said R^{1a} and R^{1b} are selected from methyl, then said R⁴ is selected from ethyl, butyl, or 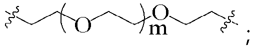 said R⁵ is selected from 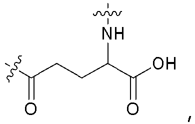 or 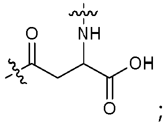
said R⁶ is selected from a C₁₅₋₂₀ fatty acid.

As a preferable technical solution of the present invention, m is selected from 0 or 1; n and p are selected from 1;
said R^{1a} and R^{1b} are independently selected from hydrogen, methyl, or 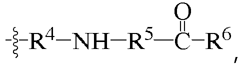 said R^{1a} and R^{1b} are not simultaneously hydrogen or methyl;
said R^{1c} is selected from hydrogen, or methyl;
said R⁴ is selected from ethyl, butyl, or 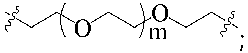
said R⁵ is selected from 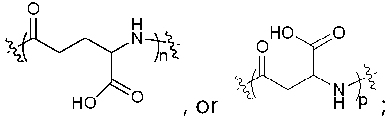
said R⁶ is selected from a C₁₅₋₂₀ fatty acid.

As a preferable technical solution of the present invention, m is selected from 0 or 1; n and p are selected from 1;
said X and Y are independently selected from 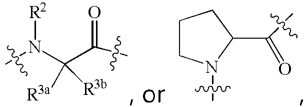 one of said X and Y is selected from 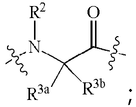
said R² is selected from hydrogen, methyl, or 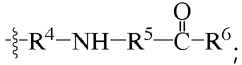
said R^{3a}, R^{3b} are each independently selected from hydrogen, methyl, or 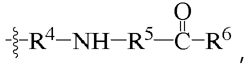 wherein, one of said R², R^{3a}, and R^{3b} is selected from 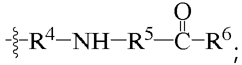
said R⁴ is selected from ethyl, butyl, or 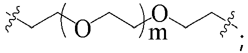
said R⁵ is selected from 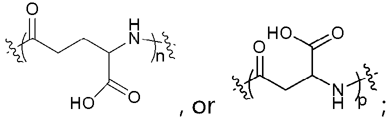
said R⁶ is selected from a C₁₅₋₂₀ fatty acid.

As a preferable technical solution of the present invention, said GLP-1 compound is selected from the compounds shown in Table 1.

As a preferable technical solution of the present invention, at least one hydrogen atom of said GLP-1 compound, or an isomer thereof, or a racemate thereof, or a pharmaceutically acceptable salt thereof is substituted by an isotope deuterium.

As a preferable technical solution of the present invention, said pharmaceutically acceptable salt is prepared from said GLP-1 compound, or an isomer thereof, or a racemate thereof, or a pharmaceutically acceptable salt thereof, with a pharmaceutically acceptable acid or base.

The present invention further provides a pharmaceutical composition comprising a therapeutically effective amount of said GLP-1 compound, or an isomer thereof, or a racemate thereof, or a pharmaceutically acceptable salt thereof, and a pharmaceutically acceptable carrier.

The present invention further provides use of said GLP-1 compound, or an isomer thereof, or a racemate thereof, or a pharmaceutically acceptable salt thereof, in the manufacture of a medicine, specifically, in the manufacture of a medicine for treating a disease, wherein the disease is a chronic related disease selected from diabetes, obesity, non-alcoholic fatty liver disease and non-alcoholic steatohepatitis, cardiovascular disease, neurodegenerative disorders, chronic kidney disease, diabetic nephropathy, peripheral arterial disease, and/or heart failure.

For clarity, general terms used in the description of the compounds are defined herein.

Unless otherwise stated, the following terms and phrases used herein are intended to have the following meanings. A specific term or phrase should not be considered indefinite or unclear without special definition, but should be understood according to its ordinary meaning. When a product name appears herein, it is intended to refer to the corresponding commercial product or its active ingredient. The term "pharmaceutically acceptable" as used herein refers to those compounds, materials, compositions and/or dosage forms that, within the scope of reliable medical judgment, are suitable for contact with tissues of humans and animals, without excessive toxicity, irritation, allergic response, or other problems or complications, and commensurate with a reasonable benefit/risk ratio.

The term "compound" as used herein refers to a molecular entity. Therefore, the term "compound" may have different structural elements in addition to the minimal elements defined for each compound or each group of compounds. The term compound can be used interchangeably with the term "construct". The term "compound" can be used to describe a prodrug in the present invention. The compound in the present invention may be referred to as "compound", and the term "compound" is also intended to encompass pharmaceutically relevant forms thereof, i.e., the present invention relates to the compounds defined herein or pharmaceutically acceptable salts, amides, or esters thereof.

The term "pharmaceutically acceptable salt" refers to a salt of a compound of the present invention, prepared from the compound found in the present invention having specific substituent(s) with a pharmaceutically acceptable acid or base.

Certain compounds of the present invention may exist in an unsolvated or a solvated form, including a hydrated form. Generally, a solvated form is equivalent to an unsolvated form and both of them are included within the scope of the present invention.

The compound of the present invention may exist in a specific geometric or stereoisomeric form. The present invention contemplates all such compounds, including cis and trans isomers, (-)- and (+)- enantiomers, (R)- and (S)- enantiomers, diastereomers, (D)-isomers, (L)-isomers, atropisomers, and their racemic mixtures and other mixtures thereof, such as enantiomerically or diastereomerically enriched mixtures, all of which are encompassed within the scope of the present invention. Additional asymmetric carbon atoms may exist in substituents such as alkyl groups. All such isomers, as well as mixtures thereof, are included within the scope of the present invention.

Optically active (R)- and (S)- isomers, as well as D and L isomers, atropisomers, etc., can be prepared by chiral synthesis or chiral reagents or other conventional techniques. If one enantiomer of a compound of the present invention is desired, it can be prepared by asymmetric synthesis or by derivation with a chiral auxiliary, wherein the resulting diastereomeric mixture is separated, and the auxiliary group is cleaved to provide the pure desired enantiomer. Alternatively, when the molecule contains a basic functional group (such as an amino group) or an acidic functional group (such as a carboxyl group), salts of diastereomers are formed with an appropriate optically active acid or base, followed by resolution of the diastereomers by conventional methods well known in the art, and then recovery to obtain the pure enantiomer. In addition, the separation of an enantiomer and a diastereomer is usually accomplished using chromatography, which employs a chiral stationary phase, optionally combined with chemical derivatization (e.g., generating carbamates from amines).

The atom(s) of the compound molecules of the present invention may be isotope(s). Isotopic derivatization can often lead to effects such as prolonged half-life, reduced clearance, metabolic stability, and increased *in vivo* activity. Moreover, also included is an embodiment wherein at least one atom is replaced by an atom having the same atomic number (proton number) and a different mass number (sum of protons and neutrons). Examples of isotopes included in the compounds of the present invention include a hydrogen atom, a carbon atom, a nitrogen atom, an oxygen atom, a phosphorus atom, a sulfur atom, a fluorine atom, a chlorine atom, which include ²H, ³H, ¹³C, ¹⁴C, ¹⁵N, ¹⁷O, ¹⁸O, ³¹P, ³²P, ³⁵S, ¹⁸F, ³⁶Cl, respectively. In particular, a radioactive isotope that emits radiation upon decay, such as ³H or ¹⁴C, can be used for drug formulations or topographical anatomy examination of compounds *in vivo.* Stable isotopes neither decay nor change in quantity, and are non-radioactive, so they can be used safely. When the atoms constituting the compound molecules of the present invention are isotopes, isotopic conversion can be achieved according to general methods by replacing the reagents used in synthesis with reagents containing the corresponding isotopes.

The compounds of the present invention may contain a non-natural proportion of atomic isotopes at one or more of the atoms constituting the compound. For example, the compounds may be labeled with radioactive isotopes, such as deuterium (²H), iodine-125 (¹²⁵I) or C-14 (¹⁴C). **All** isotopic variations of the compounds of the present invention, whether radioactive or not, are encompassed within the scope of the present invention.

Further, one or more hydrogen atoms of the compounds of the present invention are replaced by the isotope deuterium (²H). After deuteration, the compounds of the present invention have effects such as prolonged half-life, reduced clearance rate, metabolic stability and improved *in vivo* activity.

The preparation methods for said isotopic derivatives generally include: phase transfer catalysis methods. For example, preferable deuteration methods employ phase transfer catalysts (e.g., tetraalkylammonium salts, NBu₄HSO₄). Using a phase transfer catalyst to exchange methylene protons of diphenylmethane compounds results in higher deuterium introduction compared to using a deuterated silane (e.g., triethyl(deutero)methylsilane) in the presence of an acid (e.g., methanesulfonic acid) or reduction with sodium borodeuteride using a Lewis acid such as aluminum trichloride.

As used herein, the term "polypeptide" or "polypeptide sequence" refers to a compound comprising a series of two or more amino acids connected to each other by an amide (or peptide) bond. The term polypeptide can be used interchangeably with the terms "peptide" and "protein".

In one embodiment, the compound of the present invention comprises a GLP-1 polypeptide. In one embodiment, the GLP-1 polypeptide has the amino acid sequence of semaglutide. In one embodiment, the compound of the present invention comprises a GLP-1 polypeptide, wherein the GLP-1 polypeptide is a GLP-1 analog, and wherein the GLP-1 analog has at most 3 amino acid changes compared to GLP-1(7-37) (SEQ ID NO:1). In one embodiment, the compound of the present invention comprises a GLP-1 polypeptide, wherein the GLP-1 polypeptide is a GLP-1 analog, and wherein the GLP-1 analog has at most 2 amino acid changes compared to GLP-1(7-37) (SEQ ID NO:1). In one embodiment, the compound of the present invention comprises a GLP-1 derivative.

The GLP-1(7 - 37) has the polypeptide sequence of HAEGTFTSDVSSYLEGQAAKEFIAWLVKGRG(SEQ ID NO:1).

In one embodiment, the GLP-1 polypeptide is semaglutide, wherein semaglutide has the structure as follows:

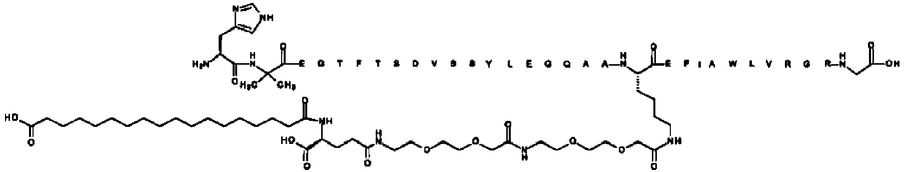

The term "GLP-1 polypeptide" as used herein refers to a polypeptide capable of binding to the GLP-1 receptor and/or activating the GLP-1 receptor. In other words, a GLP-1 polypeptide is a polypeptide having GLP-1 activity. In other words, the GLP-1 polypeptide is a GLP-1 receptor agonist. A GLP-1 polypeptide may bind and/or activate other types of receptors. That is, as long as the polypeptide binds to and/or activates the GLP-1 receptor, it qualifies as a GLP-1 polypeptide, regardless of any other receptor interactions it may be involved with. In addition to the amino acid residues responsible for GLP-1 receptor interaction, a GLP-1 polypeptide may also contain other amino acid residues that are not involved in GLP-1 receptor interaction.

The term "pharmaceutically acceptable carrier" refers to any formulation carrier or vehicle capable of delivering an effective amount of the active substance of the present invention, without interfering with the biological activity of the active substance and without toxic or side effects on the host or patient. Representative carriers include water, vegetable oil, and mineral oil, cream bases, lotion bases, ointment bases, etc. These bases include suspending agents, viscosity enhancers, transdermal penetration enhancers, etc. Their use in formulations is well known to those skilled in the art of cosmetics or topical pharmaceuticals. For further information on carriers, reference can be made to Remington: The Science and Practice of Pharmacy, 21st Ed., Lippincott, Williams & Wilkins (2005), the content of which is incorporated herein by reference.

For a drug or pharmacologically active agent, the term "effective amount" or "therapeutically effective amount" refers to an amount of the drug or agent, which is non-toxic but sufficient to achieve the desired effect. For the oral dosage forms in the present invention, the "effective amount" of one active substance in the composition refers to the amount required to achieve the desired effect when used in combination with another active substance in the composition. The determination of an effective amount varies from person to person, depends on the age and general condition of the subject, as well as on the specific active substance. An appropriate effective amount in individual cases can be determined by those skilled in the art based on routine experimentation.

"Optional" or "optionally" means that the subsequently described event or condition may but does not necessarily occur, and that the description includes instances where said event or circumstance occurs and instances where said event or circumstance does not occur.

The compounds of the present invention can be prepared by various synthesis methods well known to those skilled in the art, including the specific embodiments listed below, embodiments formed by combining them with other chemical synthesis methods, and equivalent substitutions well known to those skilled in the art. Preferred embodiments include, but are not limited to, the examples of the present invention.

### BRIEF DESCRIPTION OF FIGURES

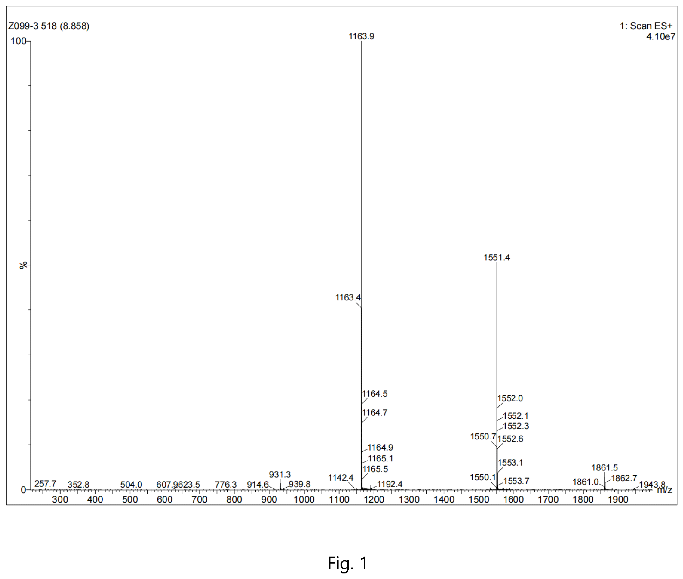

Figure 1 shows the mass spectrum (MS) of the compound of Example 2 of the present invention.

### DETAILED DESCRIPTION OF THE EMBODIMENTS

The present invention will be described in further detail below with reference to examples, but the embodiments of the present invention are not limited thereto.

### Example 1

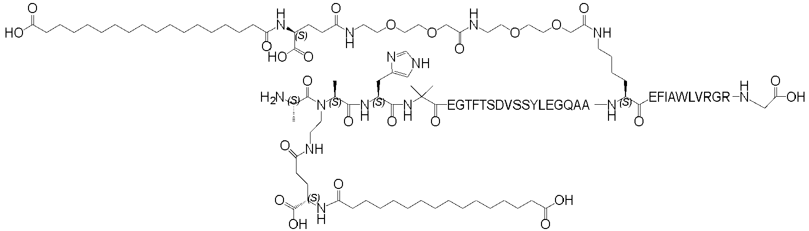

The compound of Example 1 may be prepared according to the synthetic scheme described in WO2022096636A1, the entire contents of which are incorporated herein by reference.

Specific synthetic scheme of the compound is as follows:

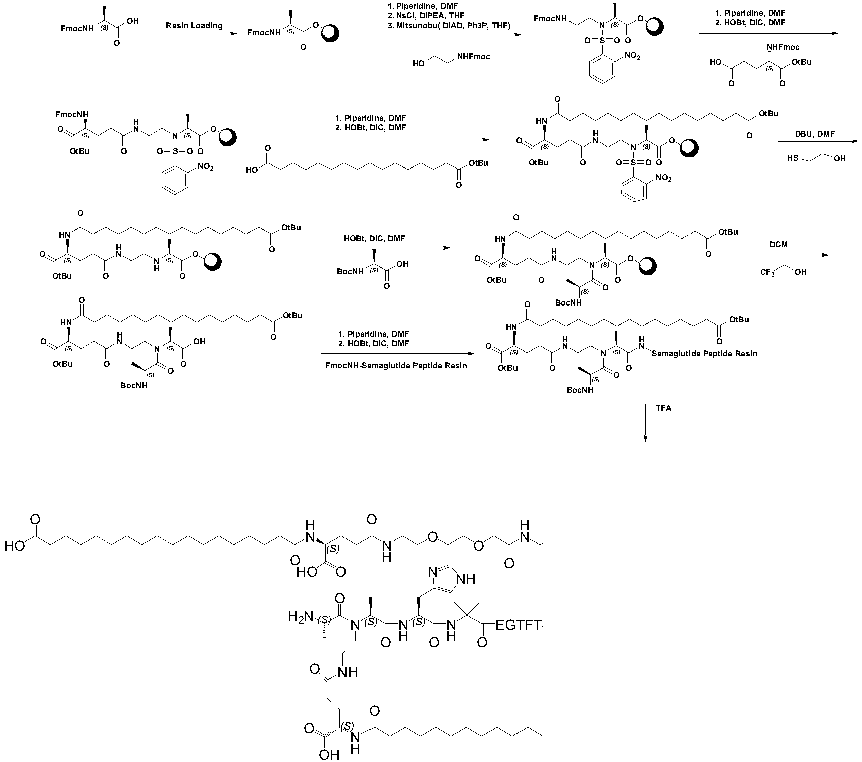

### Step 1: Synthesis of F1

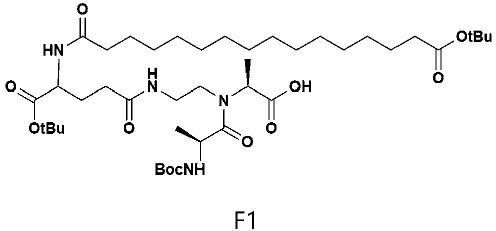

### Synthesis process description:

Compound F1 was obtained by solid-phase synthesis:
(1) Using 2-CTC resin as the carrier, first swelling it with N,N-dimethylformamide (DMF), then adding Fmoc-Ala-OH and N,N-diisopropylethylamine (DIEA) to react for several hours. After the reaction, washing the resin several times with DMF.
(2) After washing, adding methanol and DIEA separately for end capping. After end capping, washing the resin several times with DMF.
(3) Deprotecting Fmoc twice using a 20% piperidine/DMF mixed solution, for 10 minutes each time. After deprotection, washing the resin with DMF.
(4) Mixing 2-nitrobenzenesulfonyl chloride (NsCl) and DIEA in tetrahydrofuran (THF) and adding it into the resin for reaction. After the reaction, washing the resin with DMF.
(5) Mixing Fmoc-aminoethanol, diisopropyl azodicarboxylate (DIAD), and triphenylphosphine in THF separately and adding into the resin for reaction. After the reaction, washing the resin with DMF.
(6) Repeating the above step (3). to remove the Fmoc protecting group.
(7) Weighing Fmoc-Glu-OtBu and hydroxybenzotriazole (HOBt), dissolving them in DMF, then adding N,N'-diisopropylcarbodiimide (DIC) and mixing well. After mixing well, adding into the resin for reaction. After the reaction, washing the resin with DMF.
(8) Weighing mono-tert-butyl hexadecanedioate and repeating step(7). for the condensation reaction.
(9) Mixing mercaptoethanol and DBU with DMF separately, then adding into the resin to remove the Ns protecting group. After the reaction, washing the resin with DMF.
(10) Weighing Boc-Ala-OH and repeating step(7). for the condensation reaction.
(11) Washing the resin with dichloromethane and methanol separately, and drying at room temperature to obtain the peptide resin.
(12) Cleaving the resin with 20% TFE/DCM, filter, and concentrating to obtain compound F1.

### Step 2: Synthesis of F2 (semaglutide fully protected peptide resin)

Referring to the schemes described in CN200680006674.6 and WO2022096636A1 for the synthetic scheme for semaglutide.

His(Trt)-Aib-Glu(OtBu)-Gly-Thr(tBu)-Phe-Thr(tBu)-Ser(tBu)-Asp(OtBu)-Val-Ser(tBu) -Ser(tBu)-Tyr(tBu)-Leu-Glu(OtBu)-Gly-Gln(Trt)-Ala-Ala-Lys(AEEA-AEEA-γ-Glu(OtBu)-m ono-tert-butyl octadecanedioate)-Glu(OtBu)-Phe-Ile-Ala-Trp(Boc)-Leu-VaI-Arg(Pbf)-Gly-Arg(Pbf)-Gly -Wang Resin, F2

### Synthesis steps:

(1) Using Wang resin as the carrier, first swelling it with DMF. After swelling, washing the resin with DMF.
(2) Weighing Fmoc-Gly-OH, HOBt, and 4-dimethylaminopyridine (DMAP), dissolving them in DMF, then adding DIC and mixing well. After mixing, adding into the resin for reaction. After the reaction, washing the resin with DMF.
(3) After washing, adding acetic anhydride and DIEA separately for end capping. After end capping, washing the resin several times with DMF.
(4) Deprotecting Fmoc twice using a 20% piperidine/DMF mixed solution, for 10 minutes each time. After deprotection, washing the resin with DMF.
(5) Weighing Fmoc-Arg(Pbf)-OH and HOBt, adding DMF to mix and dissolve, then adding DIC to activate for 3-5 minutes. After activation, adding into the reaction vessel to start the coupling reaction; the amino acid coupling reaction lasting 1.0-3.0 hours, and the reaction endpoint is monitored by ninhydrin throughout the coupling process. After coupling, washing the resin with DMF.
(6) Repeating step(5). to couple amino acids in sequence according to the peptide sequence to obtain F2 (semaglutide fully protected peptide resin).
(7) After the reaction, washing the peptide resin with dichloromethane and methanol and air-drying at room temperature.

### Step 3: Preparation of the target compound (F1+F2)

(1) First swelling F2 (semaglutide fully protected peptide resin) with DMF. After swelling, washing the resin with DMF.
(2) Weighing F1 and HOBt, dissolving them in DMF, then adding DIC and mixing well. After mixing well, adding into the resin for reaction. After the reaction, washing the peptide resin with DMF, dichloromethane, and methanol separately, and air-drying.
(3) Preparing a cleavage solution of TFA/TIS/H₂O=95.0/2.5/2.5 v/v, mixing the cleavage solution with the resin at a volume of 10 ml of cleavage solution per gram of peptide resin. Stirring and reacting at room temperature for 2 hours. After the reaction, filtering the resin, concentrating to partially remove TFA, then adding into 8 times the volume of cleavage solution in diethyl ether to precipitate. Centrifuging to collect the crude product, and drying the crude product to constant weight.
(4) Adding the crude product to an acetonitrile/water solution and dissolving by sonication, then filtering through a 0.45 µm filter membrane. Transferring the filtered solution to HPLC rough separation for purification.
(5) HPLC rough purification: purifying the filtered sample solution according to the rough separation method and collecting qualified fractions.
(6) HPLC fine purification: purifying the qualified fractions collected from HPLC rough separation according to the HPLC fine separation method and collecting qualified fractions.
(7) Concentration and lyophilization: concentrating the qualified fractions, and filtering, dispensing, and lyophilizing the concentrated sample according to the process specifications.

The lyophilized product was sub-packaged and stored as required. Taking samples for testing, and obtaining the target compound.

### Example 2

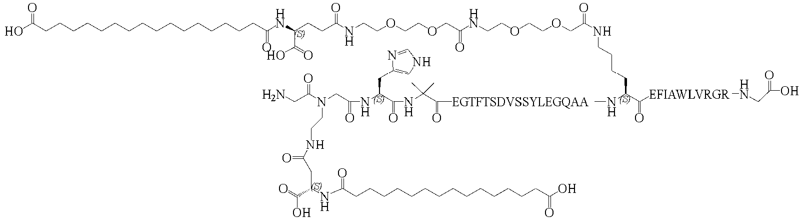

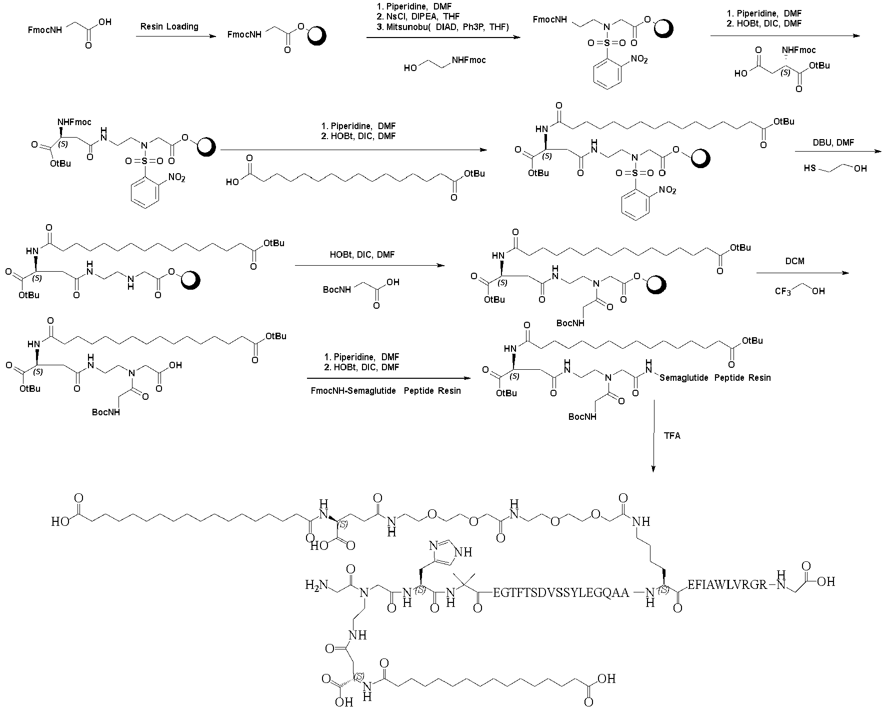

### Step 1: Synthesis of F3

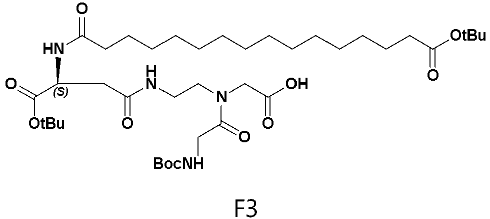

Synthesis process description: compound F3 was obtained by solid-phase synthesis:
(1) Using 2-CTC resin as the carrier, first swelling it with N,N-dimethylformamide (DMF), then adding Fmoc-Gly-OH and N,N-diisopropylethylamine (DIEA) to react for several hours. After the reaction, washing the resin several times with DMF.
(2) After washing, adding methanol and DIEA separately for end capping. After end capping, washing the resin several times with DMF.
(3) Deprotecting Fmoc twice using a 20% piperidine/DMF mixed solution, for 10 minutes each time. After deprotection, washing the resin with DMF.
(4) Mixing 2-nitrobenzenesulfonyl chloride (NsCl) and DIEA in tetrahydrofuran (THF) and adding it into the resin for reaction. After the reaction, washing the resin with DMF.
(5) Mixing Fmoc-aminoethanol, diisopropyl azodicarboxylate (DIAD), and triphenylphosphine in THF separately and adding into the resin for reaction. After the reaction, washing the resin with DMF.
(6) Repeating step(3) to remove the Fmoc protecting group.
(7) Weighing Fmoc-Asp-OtBu and hydroxybenzotriazole (HOBt), dissolving them in DMF, then adding N,N'-diisopropylcarbodiimide(DIC) and mixing well. After mixing well, adding into the resin for reaction. After the reaction, washing the resin with DMF.
(8) Weighing mono-tert-butyl hexadecanedioate and repeating step(7). for the condensation reaction.
(9) Mixing mercaptoethanol and DBU with DMF separately, then adding into the resin to remove the Ns protecting group. After the reaction, washing the resin with DMF.
(10) Weighing Boc-Gly-OH and repeating step(7). for the condensation reaction.
(11) Washing the resin with dichloromethane and methanol separately, and drying at room temperature to obtain the peptide resin.
(12) Cleaving the resin with 20% TFE/DCM, filtering, and concentrating to obtain compound F3.

### Step 2: Preparation of the target compound (F2+F3)

(1) First swelling F2 (semaglutide fully protected peptide resin) with DMF. After swelling, washing the resin with DMF.
(2) Weighing F3 and HOBt, dissolving them in DMF, then adding DIC and mixing well. After mixing, adding into the resin for reaction. After the reaction, washing the peptide resin with DMF, dichloromethane, and methanol separately, and air-drying.
(3) Preparing a cleavage solution of TFA/TIS/H₂O=95.0/2.5/2.5 v/v, mixing the cleavage solution with the resin at a volume of 10 ml of cleavage solution per gram of peptide resin. Stirring and reacting at room temperature for 2 hours. After the reaction, filtering the resin, concentrating to partially remove TFA, then adding to 8 times the volume of cleavage solution in diethyl ether to precipitate. Centrifuging to collect the crude product, and drying the crude product to constant weight.
(4) Adding the crude product to an acetonitrile/water solution and dissolving by sonication, then filtering through a 0.45 µm filter membrane. Transferring the filtered solution to HPLC rough separation for purification.
(5) HPLC rough purification: purifying the filtered sample solution according to the rough separation method and collecting qualified fractions.
(6) HPLC fine purification: purifying the qualified fractions collected from HPLC rough separation according to the HPLC fine separation method and collecting qualified fractions.
(7) Concentration and lyophilization: concentrating the qualified fractions, and filtering, dispensing, and lyophilizing the concentrated sample according to the process specifications.

The lyophilized product was sub-packaged and stored as required. Taking samples for testing, and obtaining the target compound. The mass spectrum of the compound of Example 2 is shown in Figure 1.

### Example 5

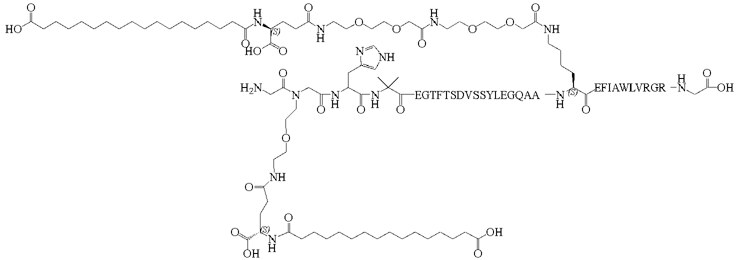

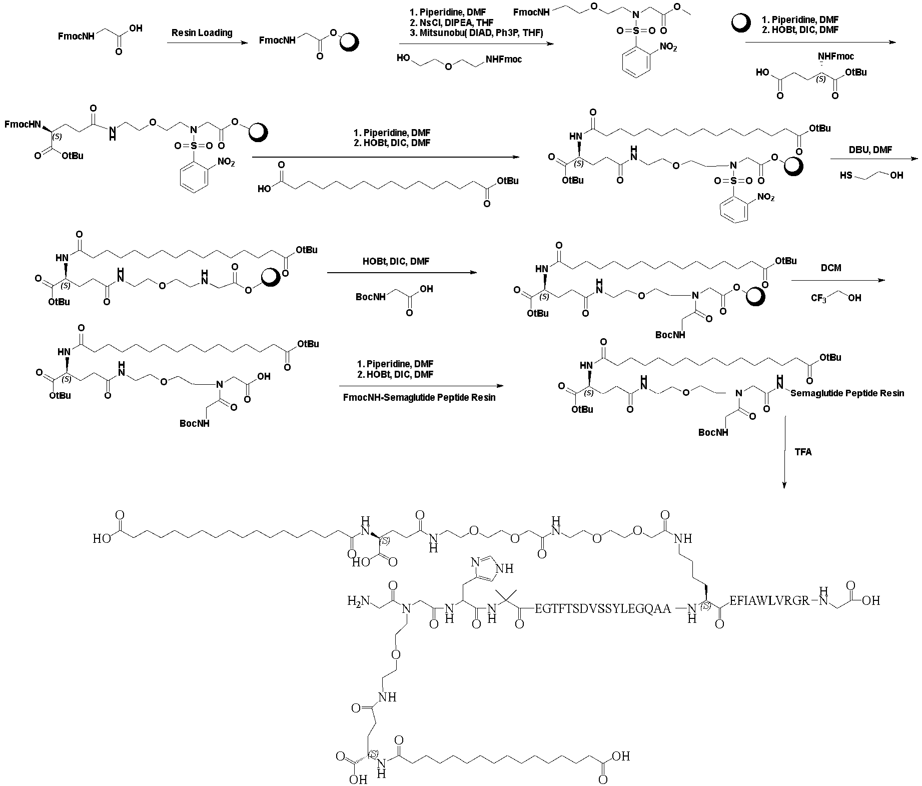

### Step 1: Synthesis of F4

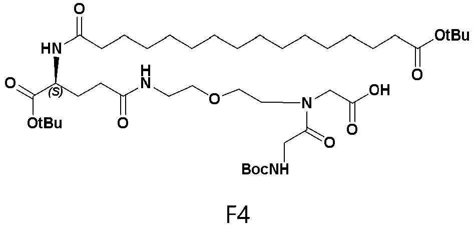

Synthesis process description: compound F4 was obtained by solid-phase synthesis:
(1) Using 2-CTC resin as the carrier, first swelling it with N,N-dimethylformamide (DMF), then adding Fmoc-Gly-OH and N,N-diisopropylethylamine (DIEA) to react for several hours. After the reaction, washing the resin several times with DMF.
(2) After washing, adding methanol and DIEA separately for end capping. After end capping, washing the resin several times with DMF.
(3) Deprotecting Fmoc twice using a 20% piperidine/DMF mixed solution, for 10 minutes each time. After deprotection, washing the resin with DMF.
(4) Mixing 2-nitrobenzenesulfonyl chloride (NsCl) and DIEA in tetrahydrofuran (THF) and adding it into the resin for reaction. After the reaction, washing the resin with DMF.
(5) Mixing Fmoc-aminoethanol, diisopropyl azodicarboxylate (DIAD), and triphenylphosphine in THF separately and adding into the resin for reaction. After the reaction, washing the resin with DMF.
(6) Repeating step(3). to remove the Fmoc protecting group.
(7) Weighing Fmoc-Glu-OtBu and hydroxybenzotriazole (HOBt), dissolving them in DMF, then adding N,N'-diisopropylcarbodiimide(DIC) and mixing well. After mixing well, adding into the resin for reaction. After the reaction, washing the resin with DMF.
(8) Weighing mono-tert-butyl hexadecanedioate and repeating step(7). for the condensation reaction.
(9) Mixing mercaptoethanol and DBU with DMF separately, then adding into the resin to remove the Ns protecting group. After the reaction, washing the resin with DMF.
(10) Weighing Boc-Gly-OH and repeating step(7). for the condensation reaction.
(11) Washing the resin with dichloromethane and methanol separately, and drying at room temperature to obtain the peptide resin.
(12) Cleaving the resin with 20% TFE/DCM, filtering, and concentrating to obtain compound F4.

### Step 2: Preparation of the target compound (F2+F4)

(1) First swelling F2 (semaglutide fully protected peptide resin) with DMF. After swelling, washing the resin with DMF.
(2) Weighing F4 and HOBt, dissolving them in DMF, then adding DIC and mixing well. After mixing well, adding into the resin for reaction. After the reaction, washing the peptide resin with DMF, dichloromethane, and methanol separately, and air-drying.
(3) Preparing a cleavage solution of TFA/TIS/H₂O=95.0/2.5/2.5 v/v, mixing the cleavage solution with the resin at a volume of 10 ml of cleavage solution per gram of peptide resin. Stirring and reacting at room temperature for 2 hours. After the reaction, filtering the resin, concentrating to partially remove TFA, then adding to 8 times the volume of cleavage solution in diethyl ether to precipitate. Centrifuging to collect the crude product, and drying the crude product to constant weight.
(4) Adding the crude product to an acetonitrile/water solution and dissolving by sonication, then filtering through a 0.45 µm filter membrane. Transferring the filtered solution to HPLC rough separation for purification.
(5) HPLC rough purification: purifying the filtered sample solution according to the rough separation method and collecting qualified fractions.
(6) HPLC fine purification: purifying the qualified fractions collected from HPLC rough separation according to the HPLC fine separation method and collecting qualified fractions.
(7) Concentration and lyophilization: concentrating the qualified fractions, and filtering, dispensing, and lyophilizing the concentrated sample according to the process specifications.

The lyophilized product was sub-packaged and stored as required. Taking samples for testing, and obtaining the target compound.

### Example 6

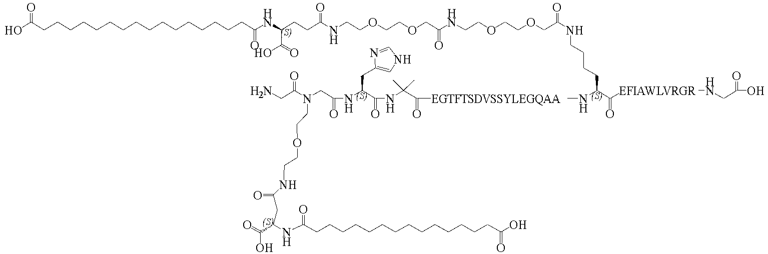

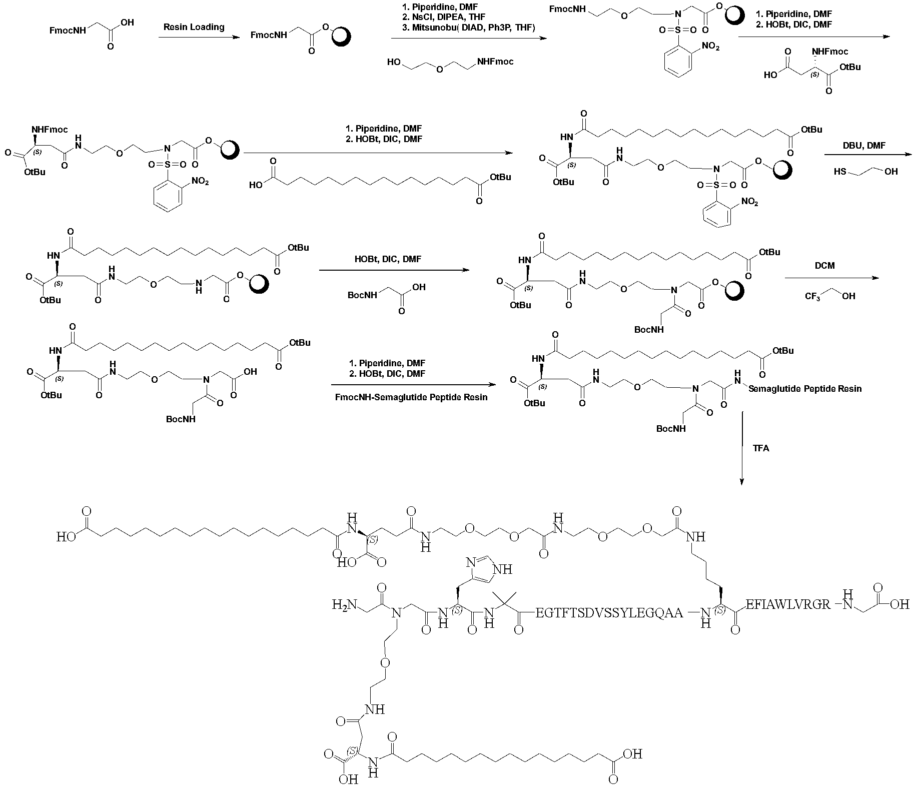

### Step 1: Synthesis of F5

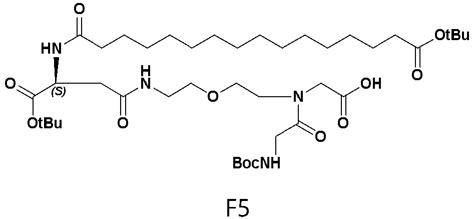

Synthesis process description: compound F5 was obtained by solid-phase synthesis:
(1) Using 2-CTC resin as the carrier, first swelling it with N,N-dimethylformamide (DMF), then adding Fmoc-Gly-OH and N,N-diisopropylethylamine (DIEA) to react for several hours. After the reaction, washing the resin several times with DMF.
(2) After washing, adding methanol and DIEA separately for end capping. After end capping is completed, washing the resin several times with DMF.
(3) Deprotecting Fmoc twice using a 20% piperidine/DMF mixed solution, for 10 minutes each time. After deprotection, washing the resin with DMF.
(4) Mixing 2-nitrobenzenesulfonyl chloride (NsCl) and DIEA in tetrahydrofuran (THF) and adding it into the resin for reaction. After the reaction, washing the resin with DMF.
(5) Mixing Fmoc-aminoethanol, diisopropyl azodicarboxylate (DIAD), and triphenylphosphine in THF separately and adding into the resin for reaction. After the reaction, washing the resin with DMF.
(6) Repeating step(3). to remove the Fmoc protecting group.
(7) Weighing Fmoc-Asp-OtBu and hydroxybenzotriazole (HOBt), dissolving them in DMF, then adding N,N'-diisopropylcarbodiimide (DIC) and mixing well. After mixing well, adding into the resin for reaction. After the reaction, washing the resin with DMF.
(8) Weighing mono-tert-butyl hexadecanedioate and repeating step(7). for the condensation reaction.
(9) Mixing mercaptoethanol and DBU with DMF separately, then adding into the resin to remove the Ns protecting group. After the reaction, washing the resin with DMF.
(10) Weighing Boc-Gly-OH and repeating step(7). for the condensation reaction.
(11) Washing the resin with dichloromethane and methanol separately, and drying at room temperature to obtain the peptide resin.
(12) Cleaving the resin with 20% TFE/DCM, filtering, and concentrating to obtain compound F5.

### Step 2: Preparation of the target compound (F2+F5)

(1) First swelling F2 (semaglutide fully protected peptide resin) with DMF. After swelling, washing the resin with DMF.
(2) Weighing F5 and HOBt, dissolving them in DMF, then adding DIC and mixing well. After mixing well, adding into the resin for reaction. After the reaction, washing the peptide resin with DMF, dichloromethane, and methanol separately, and air-drying.
(3) Preparing a cleavage solution of TFA/TIS/H₂O=95.0/2.5/2.5 v/v, mixing the cleavage solution with the resin at a volume of 10 ml of cleavage solution per gram of peptide resin. Stirring and reacting at room temperature for 2 hours. After the reaction, filtering the resin, concentrating to partially remove TFA, then adding into 8 times the volume of cleavage solution in diethyl ether to precipitate. Centrifuging to collect the crude product, and drying the crude product to constant weight.
(4) Adding the crude product to an acetonitrile/water solution and dissolving by sonication, then filtering through a 0.45 µm filter membrane. Transferring the filtered solution to HPLC rough separation for purification.
(5) HPLC rough purification: purifying the filtered sample solution according to the rough separation method and collecting qualified fractions.
(6) HPLC fine purification: purifying the qualified fractions collected from HPLC rough separation according to the HPLC fine separation method and collecting qualified fractions.
(7) Concentration and lyophilization: concentrating the qualified fractions, and filtering, dispensing, and lyophilizing the concentrated sample according to the process specifications.

The lyophilized product was sub-packaged and stored as required. Taking samples for testing, and obtaining the target compound.

### Example 7

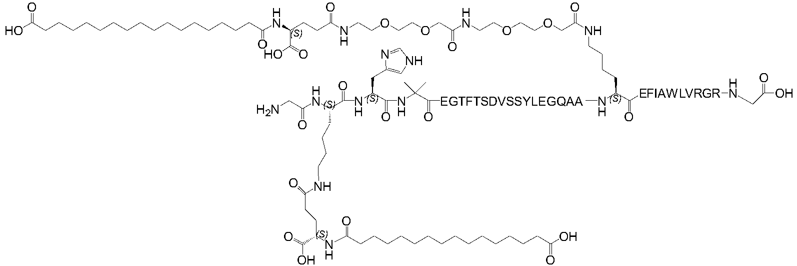

The compound of Example 7 may be prepared according to the synthetic scheme described in WO2022096636A1, the entire contents of which are incorporated herein by reference.

Specific synthetic scheme of the compound is as follows:

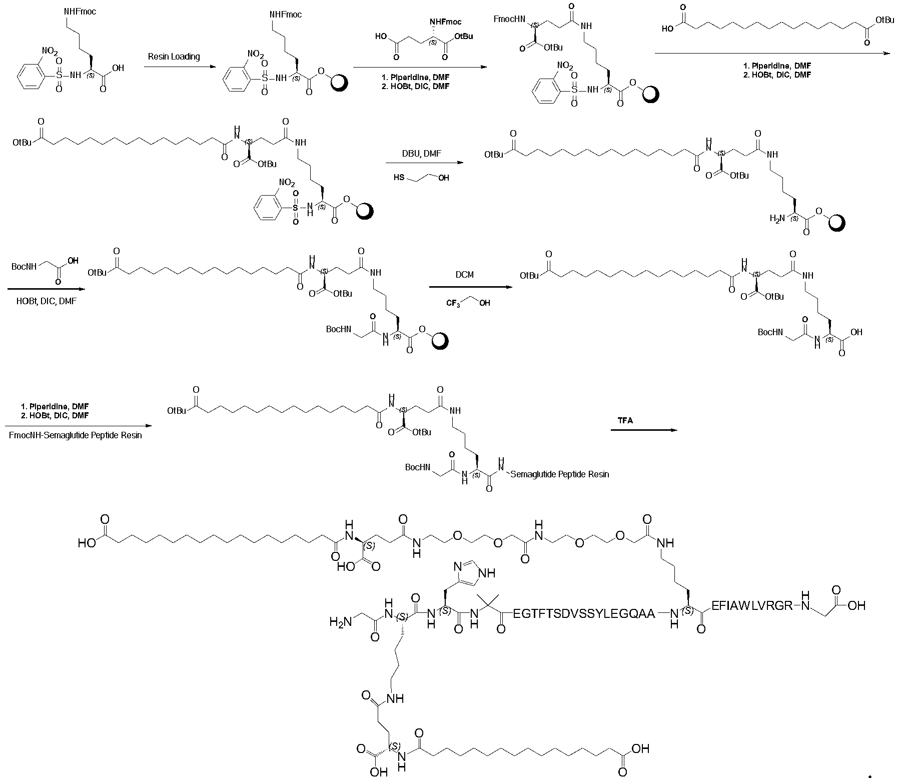

### Step 1: Synthesis of F6

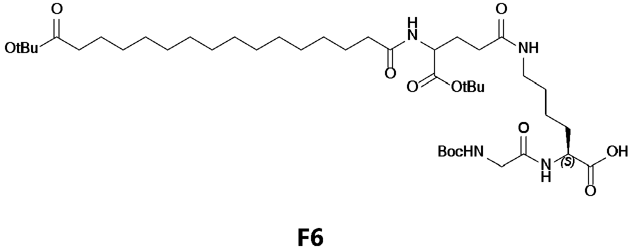

Synthesis process description: compound F6 was obtained by solid-phase synthesis:
(1) Using 2-CTC resin as the carrier, first swelling it with N,N-dimethylformamide (DMF), then adding Fmoc-Lys(o-Ns)-OH and N,N-diisopropylethylamine (DIPEA) to react for several hours. After the reaction, washing the resin several times with DMF.
(2) After washing, adding methanol and DIPEA separately for end capping. After end capping, washing the resin several times with DMF.
(3) Deprotecting Fmoc twice using a 20% piperidine/DMF mixed solution, for 10 minutes each time. After deprotection, washing the resin with DMF.
(4) Weighing Fmoc-Glu-OtBu and hydroxybenzotriazole(HOBt), dissolving them in DMF, then adding N,N'-diisopropylcarbodiimide (DIC) and mixing. After mixing, adding into the resin for reaction. After the reaction, washing the resin with DMF.
(5) Weighing mono-tert-butyl hexadecanedioate and hydroxybenzotriazole (HOBt), dissolving them in DMF, then adding N,N'-diisopropylcarbodiimide (DIC) and mixing well. After mixing well, adding into the resin for reaction. After the reaction, washing the resin with DMF.
(6) Mixing mercaptoethanol and DBU with DMF separately, then adding into the resin to remove the Ns protecting group. After the reaction, washing the resin with DMF.
(7) Weighing Boc-Gly-OH and repeating step(4). for the condensation reaction.
(8) Washing the resin with dichloromethane and methanol separately, and drying at room temperature to obtain the peptide resin.
(9) Cleaving the resin with 20% TFE/DCM, filtering, and concentrating to obtain compound F6.

### Step 2: Preparation of the target compound (F2+F6)

(1) First swelling F2 (semaglutide fully protected peptide resin) with DMF. After swelling, washing the resin with DMF.
(2) Weighing F6 and HOBt, dissolving them in DMF, then adding DIC and mixing. After mixing, adding into the resin for reaction. After the reaction, washing the peptide resin with DMF, dichloromethane, and methanol separately, and air-drying.
(3) Preparing a cleavage solution of TFA/TIS/H₂O=95.0/2.5/2.5 v/v, mixing the cleavage solution with the resin at a volume of 10 ml of cleavage solution per gram of peptide resin. Stirring and reacting at room temperature for 2 hours. After the reaction, filtering the resin, concentrating to partially remove TFA, then adding to 8 times the volume of cleavage solution in diethyl ether to precipitate. Centrifuging to collect the crude product, and drying the crude product to constant weight.
(4) Adding the crude product to an acetonitrile/water solution and dissolving by sonication, then filtering through a 0.45 µm filter membrane. Transferring the filtered solution to HPLC rough separation for purification.
(5) HPLC rough purification: purifying the filtered sample solution according to the rough separation method and collecting qualified fractions.
(6) HPLC fine purification: purifying the qualified fractions collected from HPLC rough separation according to the HPLC fine separation method and collect qualified fractions.
(7) Concentration and lyophilization: concentrating the qualified fractions, and filtering, dispensing, and lyophilizing the concentrated sample according to the process specifications.

The lyophilized product was sub-packaged and stored as required. Taking samples for testing, and obtaining the target compound.

### Example 15

The compound of Example 15 may be prepared according to the synthetic scheme described in WO2022096636A1, the entire contents of which are incorporated herein by reference.

Specific synthetic scheme of the compound is as follows:

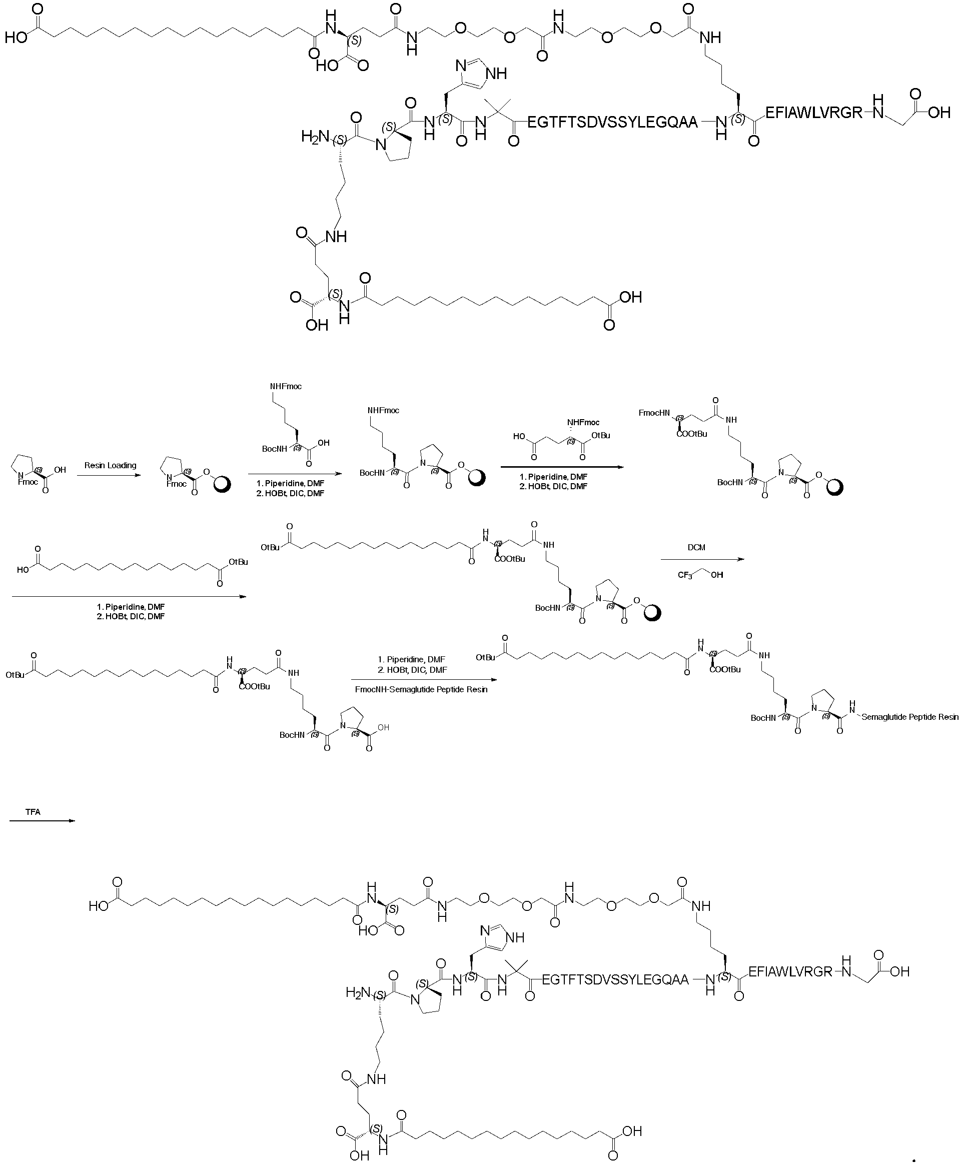

### Step 1: Synthesis of F7

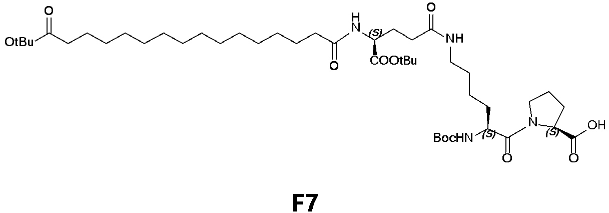

Synthesis process description: compound F7 was obtained by solid-phase synthesis:
(1) Using 2-CTC resin as the carrier, first swelling it with N,N-dimethylformamide (DMF), then adding Fmoc-Pro-OH and N,N-diisopropylethylamine (DIPEA) to react for several hours. After the reaction, washing the resin several times with DMF.
(2) After washing, adding methanol and DIPEA separately for end capping. After end capping, washing the resin several times with DMF.
(3) Deprotecting Fmoc twice using a 20% piperidine/DMF mixed solution, for 10 minutes each time. After deprotection, washing the resin with DMF.
(4) Weighing Fmoc-Lys(Boc)-OH and hydroxybenzotriazole (HOBt), dissolving them in DMF, then adding N,N'-diisopropylcarbodiimide (DIC) and mixing well. After mixing, adding into the resin for reaction. After the reaction, washing the resin with DMF.
(5) Weighing Fmoc-Glu-OtBu and hydroxybenzotriazole (HOBt), dissolving them in DMF, then adding N,N'-diisopropylcarbodiimide (DIC) and mixing. After mixing, adding into the resin for reaction. After the reaction, washing the resin with DMF.
(6) Weighing mono-tert-butyl hexadecanedioate and hydroxybenzotriazole (HOBt), dissolving them in DMF, then adding N,N'-diisopropylcarbodiimide (DIC) and mixing. After mixing, adding into the resin for reaction. After the reaction, washing the resin with DMF.
(7) Washing the resin with dichloromethane and methanol separately, and drying at room temperature to obtain the peptide resin.
(8) Cleaving the resin with 20% TFE/DCM, filtering, and concentrating to obtain compound F7.

### Step 2: Preparation of the target compound (F2+F7)

(1) First swelling F2 (semaglutide fully protected peptide resin) with DMF. After swelling, washing the resin with DMF.
(2) Weighing F7 and HOBt, dissolving them in DMF, then adding DIC and mixing. After mixing, adding to the resin for reaction. After the reaction, washing the peptide resin with DMF, dichloromethane, and methanol separately, and air-drying.
(3) Preparing a cleavage solution of TFA/TIS/H₂O=95.0/2.5/2.5 v/v, mixing the cleavage solution with the resin at a volume of 10 ml of cleavage solution per gram of peptide resin. Stirring and reacting at room temperature for 2 hours. After the reaction, filtering the resin, concentrating to partially remove TFA, then adding to 8 times the volume of cleavage solution in diethyl ether to precipitate. Centrifuging to collect the crude product, and drying the crude product to constant weight.
(4) Adding the crude product to an acetonitrile/water solution and dissolving by sonication, then filtering through a 0.45 µm filter membrane. Transferring the filtered solution to purification for HPLC rough separation.
(5) HPLC rough purification: purifying the filtered sample solution according to the rough separation method and collecting qualified fractions.
(6) HPLC fine purification: purifying the qualified fractions collected from HPLC rough separation according to the HPLC fine separation method and collecting qualified fractions.
(7) Concentration and lyophilization: concentrating the qualified fractions, and filtering, dispensing, and lyophilizing the concentrated sample according to the process specifications.

The lyophilized product was sub-packaged and stored as required. Taking samples for testing, and obtaining the target compound.

### Example 17

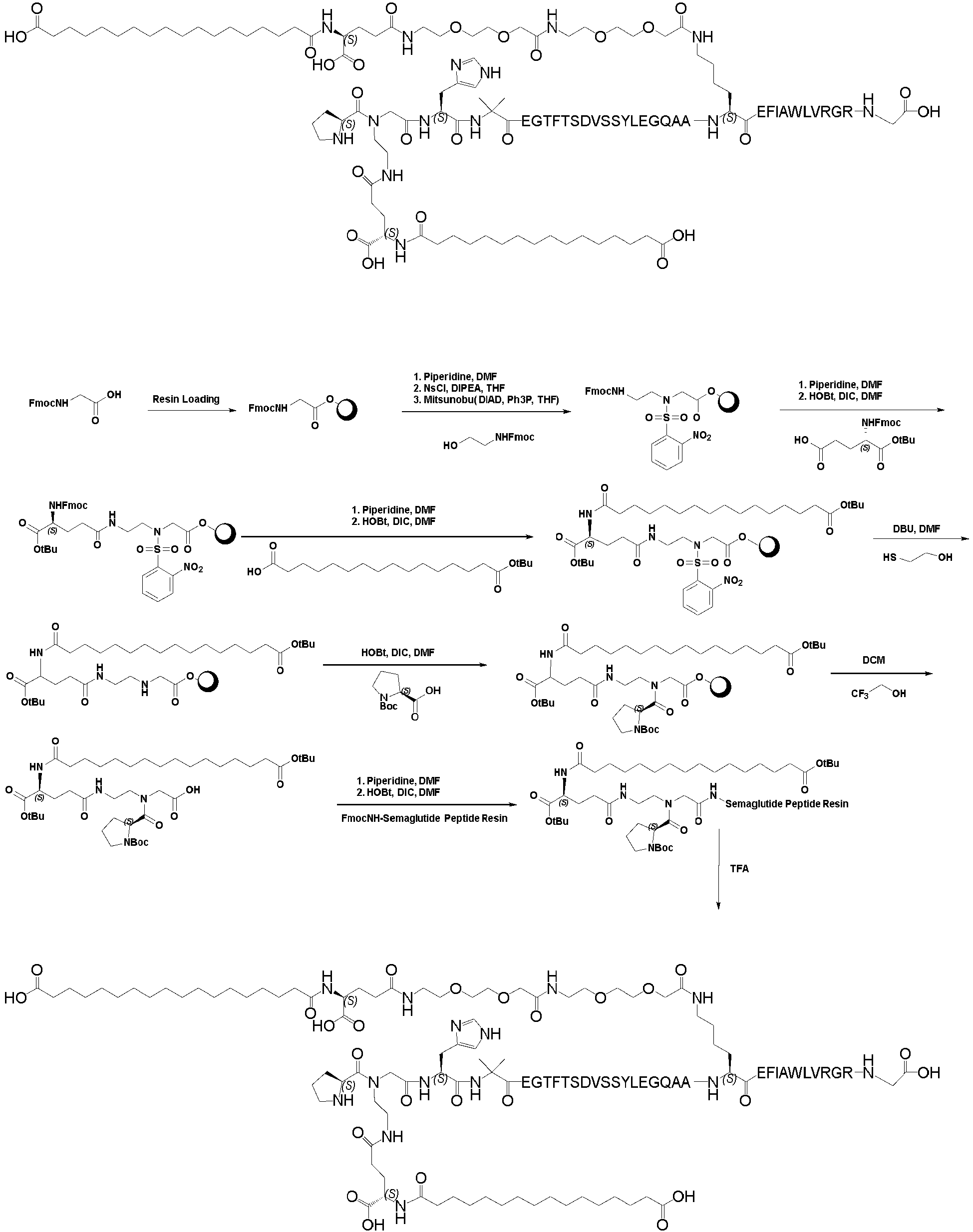

### Step 1: Synthesis of F8

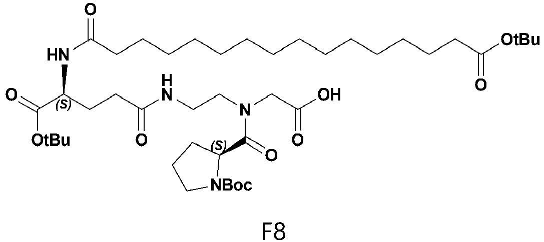

Synthesis process description: compound F8 was obtained by solid-phase synthesis:
(1) Using 2-CTC resin as the carrier, first swelling it with N,N-dimethylformamide (DMF), then adding Fmoc-Gly-OH and N,N-diisopropylethylamine (DIEA) to react for several hours. After the reaction, washing the resin several times with DMF.
(2) After washing, adding methanol and DIEA separately for end capping. After end capping, washing the resin several times with DMF.
(3) Deprotecting Fmoc twice using a 20% piperidine/DMF mixed solution, for 10 minutes each time. After deprotection, washing the resin with DMF.
(4) Mixing 2-nitrobenzenesulfonyl chloride (NsCl) and DIEA in tetrahydrofuran (THF) and adding it into the resin for reaction. After the reaction, washing the resin with DMF.
(5) Mixing Fmoc-aminoethanol, diisopropyl azodicarboxylate (DIAD), and triphenylphosphine in THF separately and adding into the resin for reaction. After the reaction, washing the resin with DMF.
(6) Repeating step(3). to remove the Fmoc protecting group.
(7) Weighing Fmoc-Glu-OtBu and hydroxybenzotriazole (HOBt), dissolving them in DMF, then adding N,N'-diisopropylcarbodiimide (DIC) and mixing. After mixing, adding to the resin for reaction. After the reaction, washing the resin with DMF.
(8) Weighing mono-tert-butyl hexadecanedioate and repeating step(7). for the condensation reaction.
(9) Mixing mercaptoethanol and DBU with DMF separately, then adding into the resin to remove the Ns protecting group. After the reaction, washing the resin with DMF.
(10) Weighing Boc-Pro-OH and repeat step(7). for the condensation reaction.
(11) Washing the resin with dichloromethane and methanol separately, and drying at room temperature to obtain the peptide resin.
(12) Cleaving the resin with 20% TFE/DCM, filter, and concentrating to obtain compound F8

### Step 2: Preparation of the target compound (F2+F8)

(1) First swelling F2 (semaglutide fully protected peptide resin) with DMF. After swelling, washing the resin with DMF.
(2) Weighing F8 and HOBt, dissolving them in DMF, then adding DIC and mixing well. After mixing well, adding into the resin for reaction. After the reaction, washing the peptide resin with DMF, dichloromethane, and methanol separately, and air-drying.
(3) Preparing a cleavage solution of TFA/TIS/H₂O=95.0/2.5/2.5 v/v, mixing the cleavage solution with the resin at a volume of 10 ml of cleavage solution per gram of peptide resin. Stirring and reacting at room temperature for 2 hours. After the reaction, filtering the resin, concentrating to partially remove TFA, then adding to 8 times the volume of cleavage solution in diethyl ether to precipitate. Centrifuging to collect the crude product, and drying the crude product to constant weight.
(4) Adding the crude product to an acetonitrile/water solution and dissolving by sonication, then filtering through a 0.45 µm filter membrane. Transferring the filtered solution to purification for HPLC rough separation.
(5) HPLC rough purification: purifying the filtered sample solution according to the rough separation method and collecting qualified fractions.
(6) HPLC fine purification: purifying the qualified fractions collected from HPLC rough separation according to the HPLC fine separation method and collecting qualified fractions.
(7) Concentration and lyophilization: concentrating the qualified fractions, and filtering, dispensing, and lyophilizing the concentrated sample according to the process specifications.

The lyophilized product was sub-packaged and stored as required. Taking samples for testing, and obtaining the target compound.

### Example 3, 4, 8-14, 16, and 18-45

The compounds of Examples 3, 4, 8-14, 16, 18-45 were prepared with reference to the synthesis schemes described in the above Examples 1, 2, 5-7, 15, 17, or with reference to the preparation methods in the patent literature WO2022096636A1. The GLP-1 compounds of the present invention are shown in Table 1.

**Table 1: GLP-1 compounds**

| No. | compounds | Calculated mass |
|---|---|---|
| 1 | 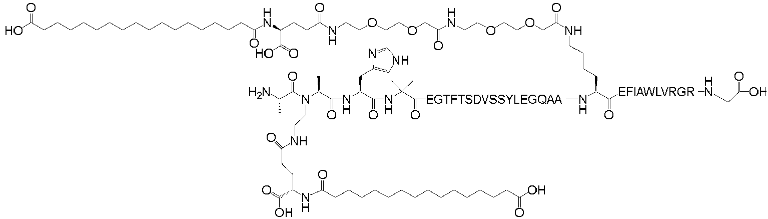 | M/3 = 1565.4 |
| | | M/4 = 1174.4 |
| | | M/5 = 940.0 |
| 2 | 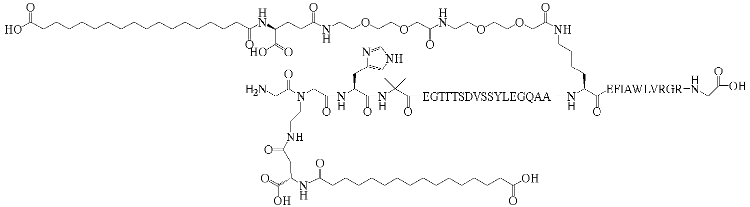 | M/3 = 1551.4 |
| | | M/4 = 1163.9 |
| 3 | 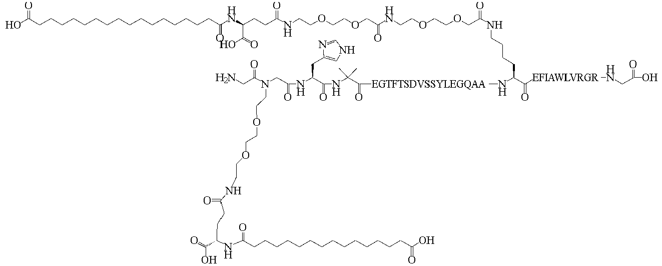 | M/3 = 1585.6 |
| | | M/4 = 1189.4 |
| | | M/5=952.0 |
| 4 | 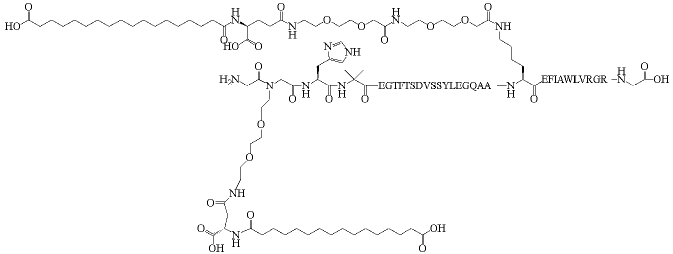 | M/3 = 1581.0 |
| | | M/4 = 1185.9 |
| | | M/5 = 948.8 |
| 5 | 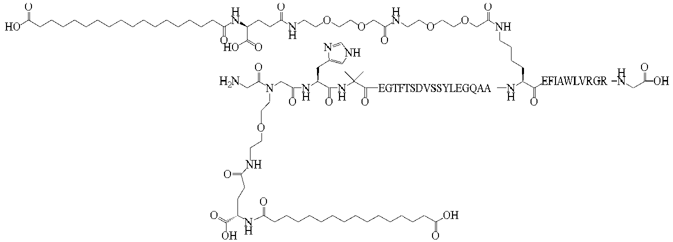 | M/3 = 1570.7 |
| | | M/4 = 1178.2 |
| | | M/5=942.8 |
| 6 | 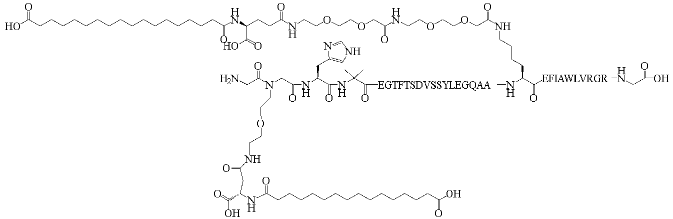 | M/3 = 1566.4 |
| | | M/4=1174.5 |
| | | M/5=940.1 |
| 7 | 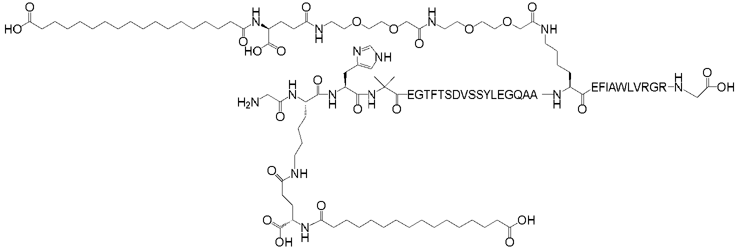 | M/3 = 1565.4 |
| | | M/4=1174.5 |
| | | M/5=939.7 |
| 8 | 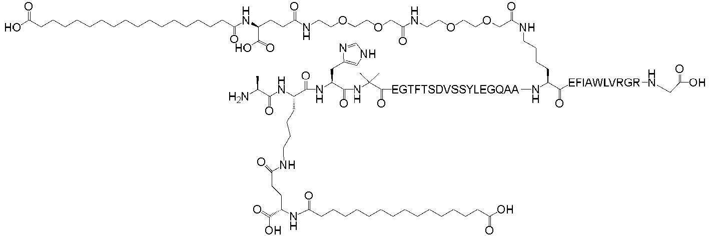 | M/3 = 1570.2 |
| | | M/4 = 1178.0 |
| | | M/5=942.5 |
| 9 | 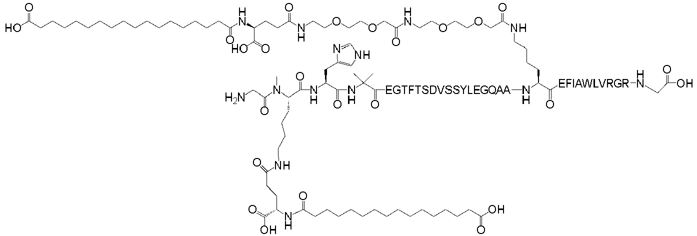 | M/3 = 1571.4 |
| | | M/4 = 1178.9 |
| | | M/5 = 943.5 |
| 10 | 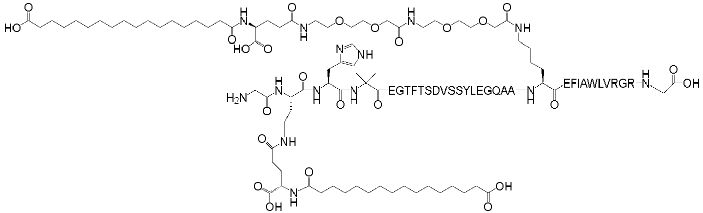 | M/3 = 1556.7 |
| | | M/4 = 1167.5 |
| | | M/5=934.0 |
| 11 | 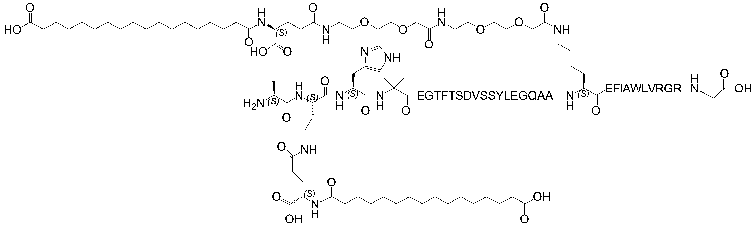 | M/3 = 1561.1 |
| | | M/4 = 1171.0 |
| | | M/5 = 937.1 |
| 12 | 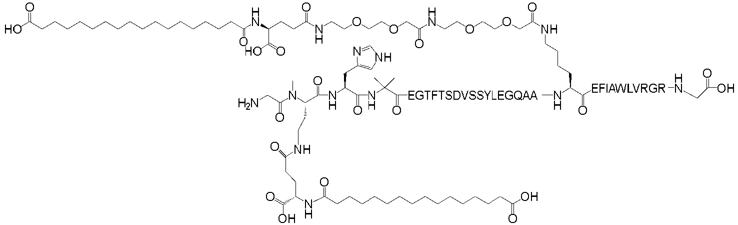 | M/3 = 1561.9 |
| | | M/4=1171.8 |
| | | M/5=937.9 |
| 13 | 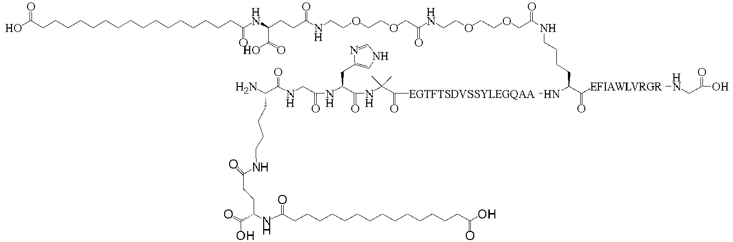 | M/3 = 1565.4 |
| | | M/4 = 1174.4 |
| | | M/5 = 940.0 |
| 14 | 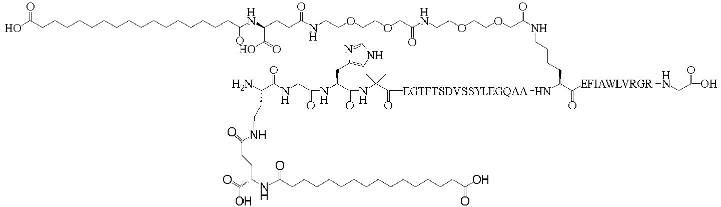 | M/3 = 1556.3 |
| | | M/4 = 1167.5 |
| | | M/5=933.9 |
| 15 | 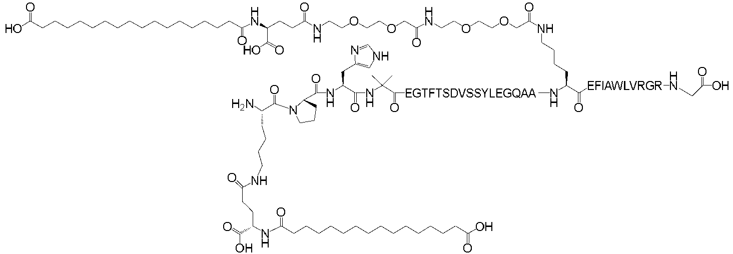 | M/3 = 1579.0 |
| | | M/4 = 1184.4 |
| | | M/5 = 948.0 |
| 16 | 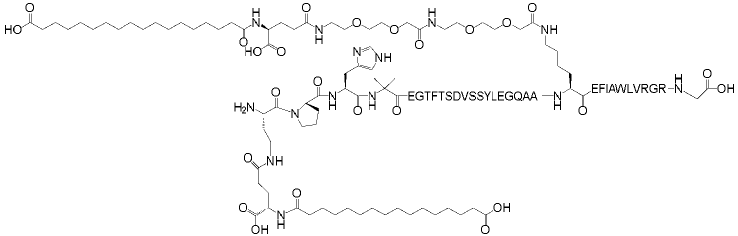 | M/3 = 1569.3 |
| | | M/4 = 1177.4 |
| | | M/5 = 942.2 |
| 17 | 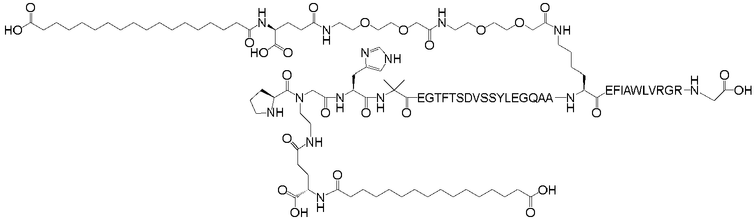 | M/3 = 1569.5 |
| | | M/4 = 1177.4 |
| | | M/5=942.2 |
| 18 | 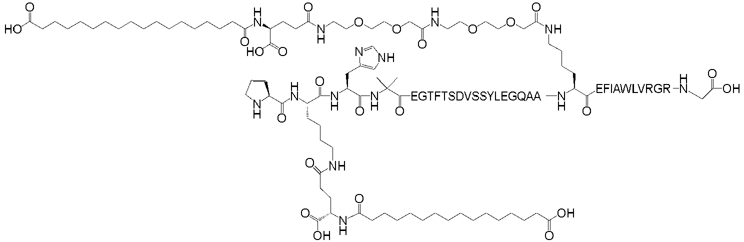 | M/3 = 1579.8 |
| | | M/4 = 1185.2 |
| | | M/5 = 948.1 |
| 19 | 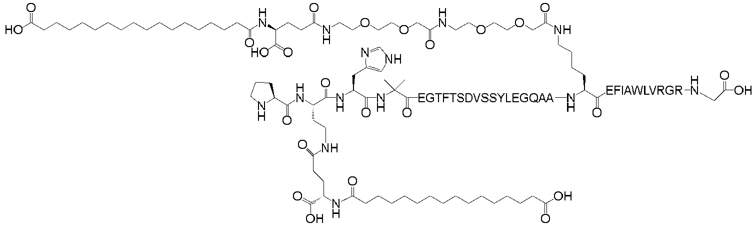 | M/3 = 1570.7 |
| | | M/4 = 1178.3 |
| | | M/5 = 942.9 |
| 20 | 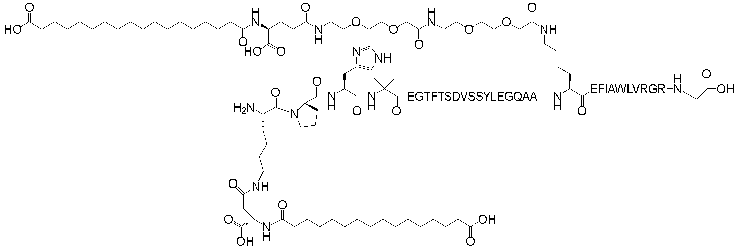 | M/3 = 1574.2 |
| | | M/4 = 1180.8 |
| | | M/5 = 944.9 |
| 21 | 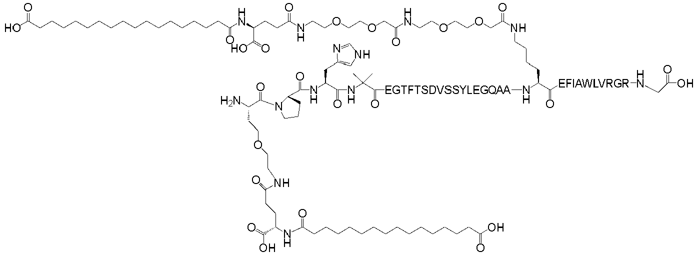 | M/3 = 1584.2 |
| | | M/4 = 1188.4 |
| | | M/5 = 951.2 |
| 22 | 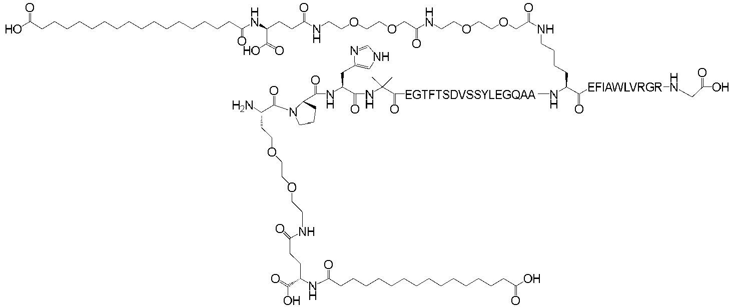 | M/3 = 1599.1 |
| | | M/4 = 1199.4 |
| | | M/5=959.6 |
| 23 | 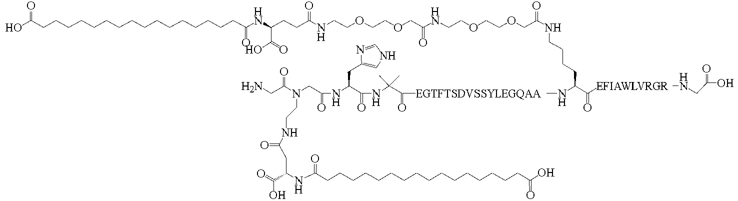 | M/3 = 1560.8 |
| | | M/4 = 1170.6 |
| | | M/5 = 936.5 |
| 24 | 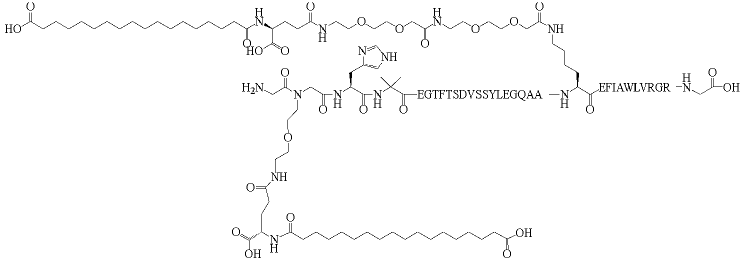 | M/3 = 1580.1 |
| | | M/4 = 1185.1 |
| | | M/5 = 948.1 |
| 25 | 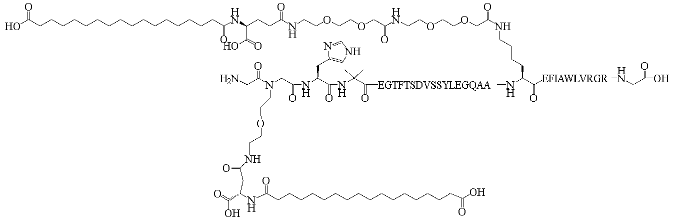 | M/3 = 1575.5 |
| | | M/4 = 1181.6 |
| | | M/5 = 945.3 |
| 26 | 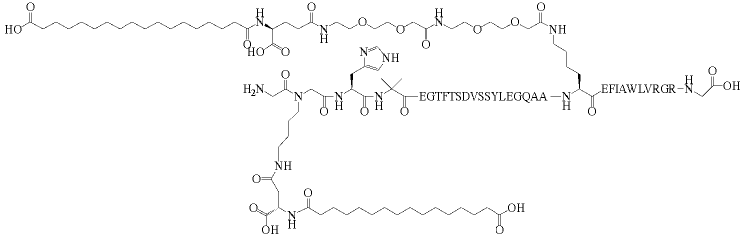 | M/3 = 1560.8 |
| | | M/4 = 1170.6 |
| | | M/5 = 936.5 |
| 27 | 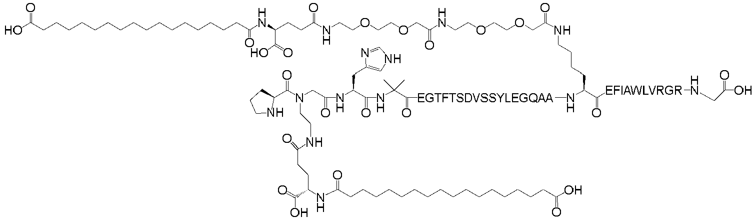 | M/3 = 1578.8 |
| | | M/4 = 1184.1 |
| | | M/5=947.3 |
| 28 | 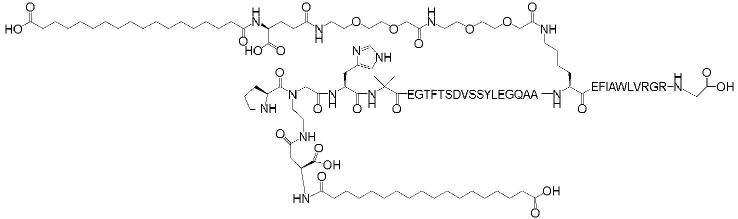 | M/3 = 1564.8 |
| | | M/4 = 1173.6 |
| | | M/5=938.9 |
| 29 | 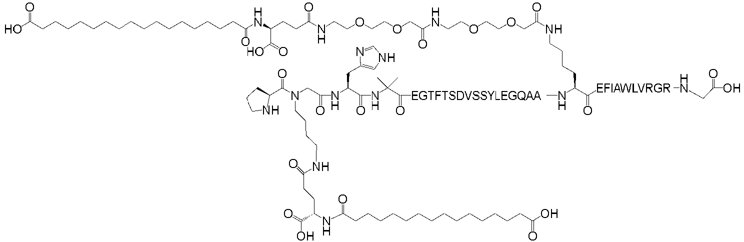 | M/3 = 1578.8 |
| | | M/4 = 1184.1 |
| | | M/5 = 947.3 |
| 30 | 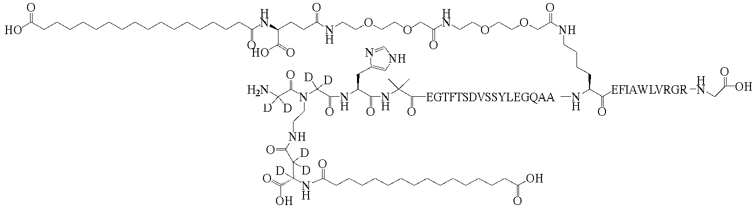 | M/3 = 1553.8 |
| | | M/4 = 1165.3 |
| | | M/5 = 932.3 |
| 31 | 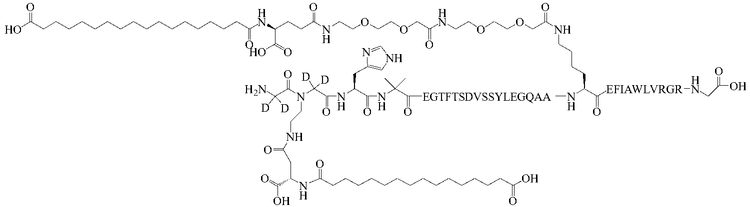 | M/3 = 1552.8 |
| | | M/4 = 1164.6 |
| | | M/5 = 931.7 |
| 32 | 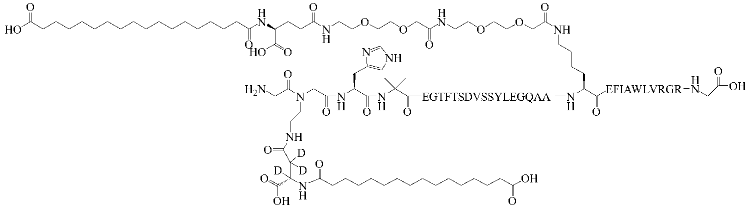 | M/3 = 1552.4 |
| | | M/4 = 1164.3 |
| | | M/5=931.5 |
| 33 | 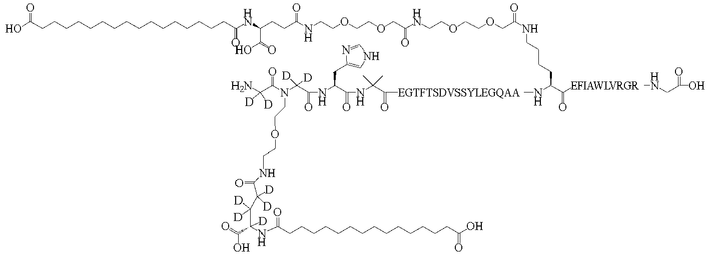 | M/3=1559.1 |
| | | M/4 = 1169.4 |
| | | M/5=935.5 |
| 34 | 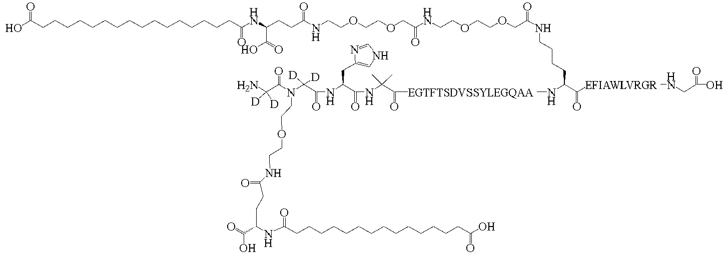 | M/3 = 1572.1 |
| | | M/4 = 1179.1 |
| | | M/5=943.3 |
| 35 | 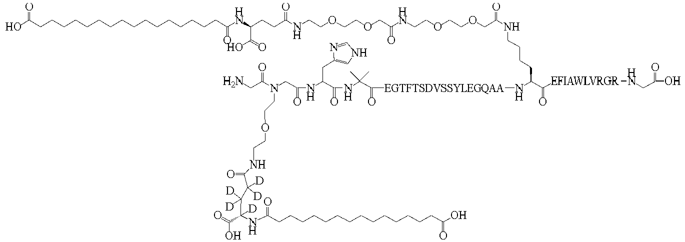 | M/3 = 1557.8 |
| | | M/4 = 1168.4 |
| | | M/5=934.7 |
| 36 | 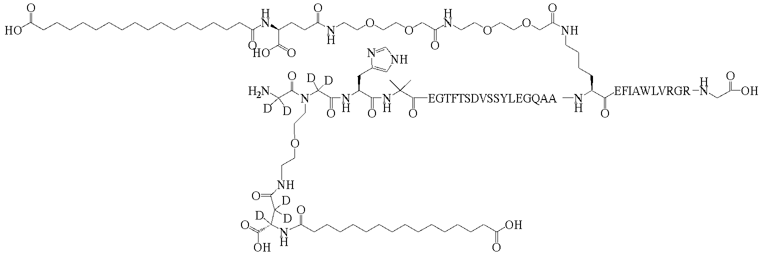 | M/3 = 1568.5 |
| | | M/4 = 1176.4 |
| | | M/5 = 941.1 |
| 37 | 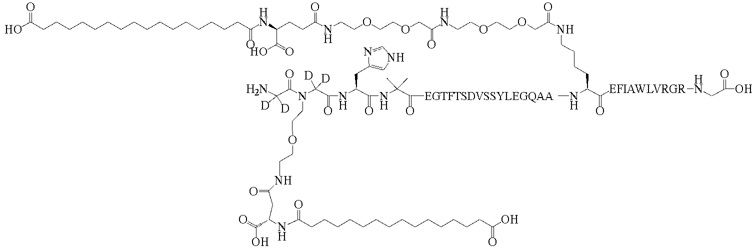 | M/3 = 1567.5 |
| | | M/4 = 1175.6 |
| | | M/5=940.5 |
| 38 | 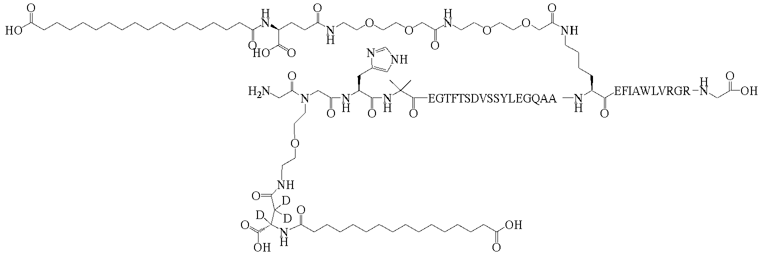 | M/3 = 1567.1 |
| | | M/4 = 1175.4 |
| | | M/5=940.3 |
| 39 | 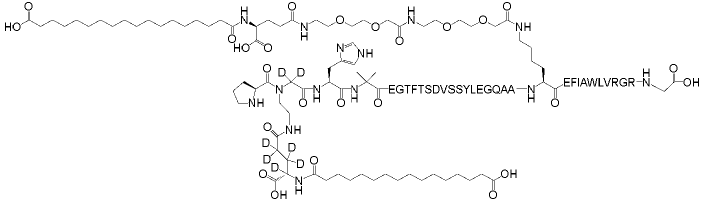 | M/3 = 1571.8 |
| | | M/4 = 1178.8 |
| | | M/5 = 943.1 |
| 40 | 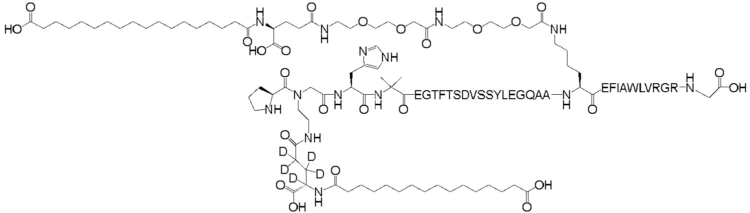 | M/3 = 1571.1 |
| | | M/4 = 1178.4 |
| | | M/5=942.7 |
| 41 | 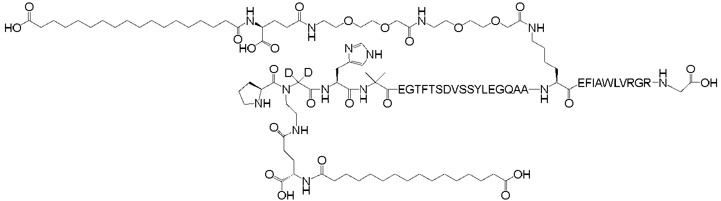 | M/3 = 1570.1 |
| | | M/4 = 1177.6 |
| | | M/5=942.1 |
| 42 | 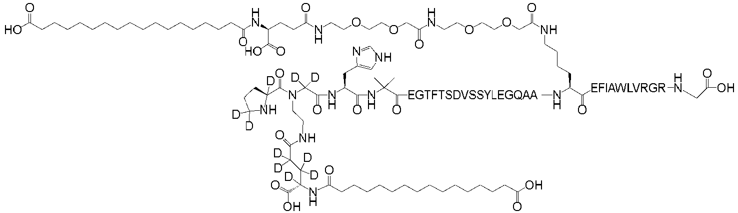 | M/3 = 1572.8 |
| | | M/4 = 1179.6 |
| | | M/5 = 943.7 |
| 43 | 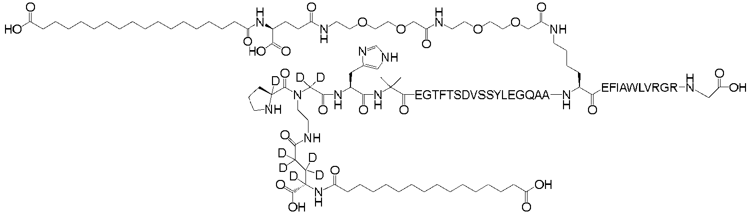 | M/3 = 1572.1 |
| | | M/4 = 1179.1 |
| | | M/5=943.3 |
| 44 | 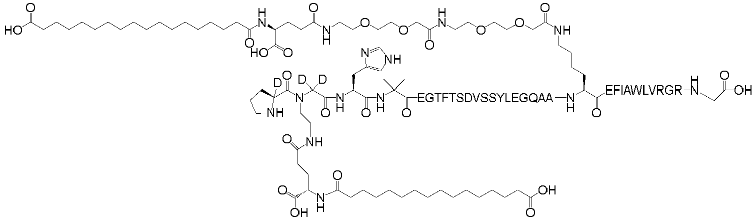 | M/3 = 1570.5 |
| | | M/4 = 1177.8 |
| | | M/5=942.3 |
| 45 | 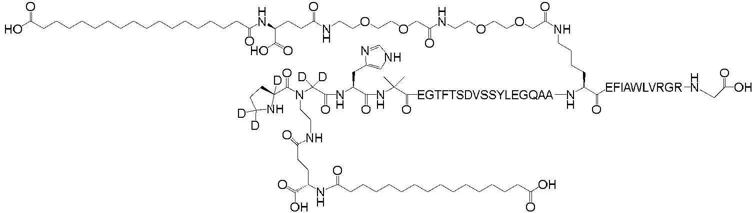 | M/3 = 1571.1 |
| | | M/4 = 1178.4 |
| | | M/5=942.7 |

### Comparative Example 1

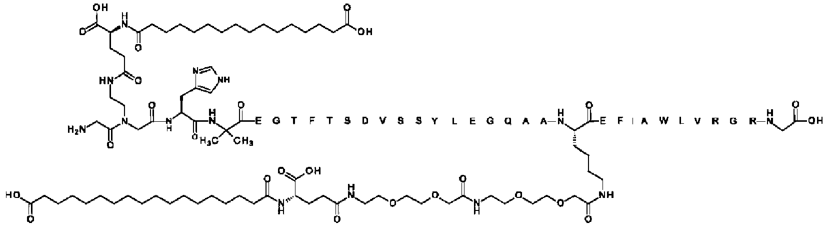

The compound of comparative Example 1 may be prepared with reference to the synthetic scheme described in Example 1 of CN202180075068.4.

### Example 46: Determination of Conversion Half-life

A peptide stock solution was prepared by dissolving the test sample in a PBS buffer to a target concentration of 200 µM, wherein the PBS buffer was Dulbecco's phosphate-buffered saline free of Ca²⁺ and Mg²⁺, pH = 7.4. The pH of the peptide stock solution was adjusted to 7.4 using HCl or NaOH. The sample was transferred to HPLC vials. The vials were capped tightly to prevent evaporation. The HPLC vials were incubated at 37°C, and samples were taken at various time points within 2 weeks, flash-frozen at -80°C, and stored at -20°C until analysis. Samples were analyzed using LC/MS or liquid chromatography. The conversion half-life of the test sample was calculated based on the reduction in the amount of the test sample prototype.

The conversion half-life of the preferable compounds of the test sample in the present invention is not less than 24 hours, indicating a long conversion half-life. Among these, more preferable compounds, for example, those from Example 2-6, Example 9, Example 12, Example 17, Example 21, and Example 22, have an *in vitro* conversion half-life of not less than 50h.

### Example 47: Determination of Terminal Half-life

The *in vivo* terminal half-life of test samples was evaluated using minipigs, with each sample tested on three minipigs. Administration was performed intravenously. The samples were prepared with a vehicle at pH 7.4, and the administration volume was 0.05 mL/kg. After administration, approximately 0.8 mL of venous blood was collected at different time points. Plasma was prepared by centrifugation at 4000 rpm for 10 minutes and then stored at -20°C until analysis. The concentration of the active ingredient was detected using LC/MS or liquid chromatography. The plasma concentration (relative to time) curves of the test compounds were evaluated using WinNonlin software through non-compartmental analysis (NCA) to calculate the pharmacokinetic parameters of terminal half-life.

The terminal half-life of the preferable compounds of the present invention after administration is not less than 30 hours.

### Example 48: GLP-1 Activity Assay

The agonistic effect of the test substance on the GLP-1 receptor was determined using a U20S-GLP-1R stable transfected cell line and a HTRF detection method.

### Experimental procedure:

1. According to the experimental procedure of the cAMP-Gs Dynamic HTRF kit, the 5x Stimulation buffer (SB) provided in the kit was diluted to 1xSB with ddH₂O. IBMX was added to the 1xSB solution to a final concentration of 500 µM to prevent degradation of cAMP. Then, a working solution of the test substance was prepared using 1xSB and subjected to a series of gradient dilutions.
2. U2OS-GLP-1R cells were digested and collected. After resuspending and counting, the cell density was diluted to 2x10⁶ cells/mL using 1xSB solution. Then, 5 µL per well was seeded into a 384-well cell culture plate, resulting in 10,000 cells per well.
3. The test substances were added at different concentrations into the corresponding cell wells at 5 µL per well, and incubated in a 37°C incubator for 30 minutes.
4. According to the steps provided by the cAMP-Gs Dynamic HTRF kit, cAMP standard solutions of different concentrations were prepared and added into the 384-well plate.
5. The cAMP d2 reagent and Eu Cryptate antibody provided in the kit were diluted to 1x using Lysis & Detection Buffer. Then, d2 and Eu solutions were added to the 384-well plate, 5 µL each well. After incubation at room temperature for 2 hours, the plate was read using the HTRF module (665/620 nm) of a microplate reader, and experimental data was collected.
6. A curve was plotted using the signal value versus the compound concentration. Curve fitting and EC₅₀ calculation were performed using nonlinear regression of GraphPad Prism software. The test results show that the preferable compounds of the present invention exhibit excellent GLP-1 activity, with EC₅₀ < 100 nM. Results are shown in Table 2.

**Table 2: GLP-1 Activity of the compounds**

| Example No. | EC₅₀(nM) | Example No. | EC₅₀(nM) |
|---|---|---|---|
| 2 | 9.3 | 3 | 15.7 |
| 4 | 16.9 | 5 | 15.0 |
| 6 | 14.3 | 9 | 35.8 |
| 12 | 36.1 | 17 | 5.7 |
| 21 | 27.7 | 22 | 53.1 |
| Comparative Example 1 | 90.3 | | |

### Example 49: Pharmacokinetic Experiment

### 1. Reagents and Instruments

Dulbecco's phosphate buffered saline (DPBS) (Batch No.: F2326001, Shanghai Aladdin Biochemical Technology Co., Ltd.). LC-MS instrument (Thermo TSQ Altis Plus).

### 2. Experimental Animals

SD rats: Male, 180-250g, purchased from Guangdong Vital River Laboratory Animal Technology Co., Ltd.

### 3. Formulation Preparation

The test sample powder was accurately weighed and completely dissolved in Dulbecco's phosphate buffered saline free of Ca²⁺/Mg²⁺, mixed well to obtain a 0.1 mg/mL solution, and administered intravenously at a dose of 2 mL/kg.

### 4. Blood Sample Collection

After intravenous administration to the rats, approximately 200 µL of venous blood was collected into EDTA-K2 anticoagulant-coated EP tubes pre-dose and post-dose at 0, 0.5, 2, 5, 24, 48, and 72 hours . The blood was centrifuged at 12,000 rpm for 2 minutes. The plasma was collected and stored frozen at -20°C until analysis.

### 5. Bioanalysis

A certain amount of the test sample was accurately weighed and dissolved in DMSO to prepare a 2 mg/mL stock solution. An appropriate amount of the compound stock solution was accurately pipetted and diluted with an acetonitrile-aqueous solution (ACN:H₂O=1:1) to prepare a series of standard solutions. A 4 µL aliquot of each standard solution mentioned above was pipetted and added 36 µL of blank plasma. The mixture was vortex-mixed to prepare plasma samples corresponding to nominal plasma concentrations of 0.3, 1, 3, 10, 30, 100, 300, 500, 1000, 3000, 5000, and 10000 ng/mL for establishing the standard curve. 30 µL of plasma was taken, to which was added 150 µL of an acetonitrile/methanol (1:1) solution containing the internal standard propranolol (5 ng/mL). After vortex mixing, the mixture was centrifuged at 4,000 rpm for 10 minutes. A 100 µL aliquot of the supernatant was taken, mixed with 100 µL of ultrapure water, and then subjected to LC-MS analysis. The LC-MS detection conditions were as follows:
Chromatographic column: YMC-Triart C18, 33*2.1mm, 5µm.

Mobile phase A: water (0.1% formic acid); Mobile phase B: acetonitrile; Flow rate: 0.5 mL/min, and the gradient elution program is shown in Table 3 below:

**Table 3**

| Time(min) | A(%) | B(%) |
|---|---|---|
| 0 | 95% | 5% |
| 0.6 | 95% | 5% |
| 1.5 | 5% | 95% |
| 2.5 | 5% | 95% |
| 2.51 | 95% | 5% |
| 3.2 | 95% | 5% |

### 6. Data Processing

After determining the plasma drug concentration by LC-MS, the pharmacokinetic parameters in rats post-administration were calculated using a non-compartmental model with WinNonlin 6.1 software. The results are shown in Table 4.

**Table 4**

| No. | Dose (mg/kg) | **AUClast (h*ng/ml)** |
|---|---|---|
| 2 | 0.2 | 101000 |
| 5 | 0.2 | 81500 |
| 6 | 0.2 | 98300 |
| 17 | 0.2 | 99300 |

As shown in Table 4, the compounds of the present invention exhibit higher exposure and are superior to the compound of comparative Example 1.

### Example 50: In Vivo Efficacy

High-fat diet-induced 16-week-old DIO (diet-induced obese) mice (purchased from Guangdong Vital River Laboratory Animal Technology Co., Ltd.) were used in the experiment, with wild-type mice of the same age as normal controls. Before the experiment, DIO mice were grouped according to body weight and randomly divided into 3 groups, 5 mice per group. Wild-type mice served as a blank control group, with a total of 5 mice. Both DIO mice and wild-type mice were administered subcutaneously, once every three days. Detailed dosing design is shown in Table 1. During the experiment, animal behavior, fur condition, water intake, and urination were observed, body weight was measured every other day, and any abnormalities were recorded. The experimental period was 15 days in total.

**Table 5: Mouse dosing groups**

| Group No. | Group | Number of Animals | Dose (mg/kg) | Dosing volume (mL/kg) | Dosing frequency | Route of Administration |
|---|---|---|---|---|---|---|
| 1 | Blank | 5 | 0 | 10 | Q3d | S.C |
| 2 | Model | 5 | 0 | 10 | Q3d | S.C |
| 3 | Control | 5 | 1 | 10 | Q3d | S.C |
| 4 | Compound of the invention | 5 | 1 | 10 | Q3d | S.C |

The results showed no abnormalities in mice from any dosing group during administration. After the final dosing, the body weight reduction rate in mice from dosing group 4 was 35.4% compared to pre-dosing, while the body weight reduction rate in mice from the control group was 21.7%. The results indicate that the compounds of the present invention have a superior weight loss effect on DIO mice compared to the control group.

The aforementioned examples are only preferable embodiments of the present invention. However, the embodiments of the present invention are not limited by the aforementioned examples. Any other changes, alterations, substitutions, combinations, or simplifications made without departing from the spirit and principle of the present invention shall be considered equivalent substitution modes and are included within the protection scope of the present invention.

## Claims

1. A GLP-1 compound shown in formula (I), or an isomer thereof, or a racemate thereof, or a pharmaceutically acceptable salt thereof, **characterized in that**, comprising: 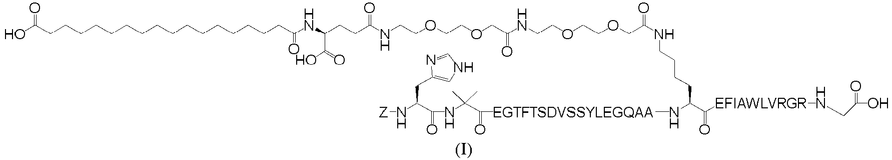
wherein, Z is selected from 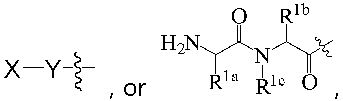 said X and Y are each independently selected from 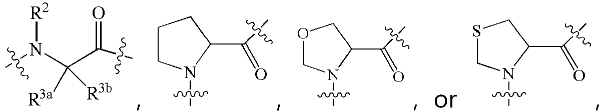 one of X and Y is selected from 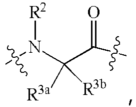
and a bond in the terminal group of X is linked to a hydrogen;
said R^{1a}, R^{1b}, R^{1c}, R², R^{3a}, R^{3b} are each independently selected from hydrogen, C₁₋₆ alkyl, C₃₋₆ cycloalkyl, or 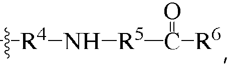 or alternatively, said R^{3a} and R^{3b} are cyclized, together with the atom to which they are attached, form a C₃₋₆ cycloalkyl; at least one of R^{1a}, R^{1b}, R^{1c}, R², R^{3a} and R^{3b} is selected from 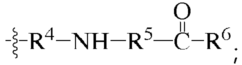
said R⁴ is selected from C₁₋₆ alkyl, or 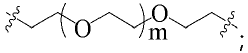
R⁵ is selected from one of 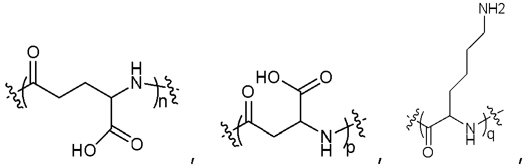 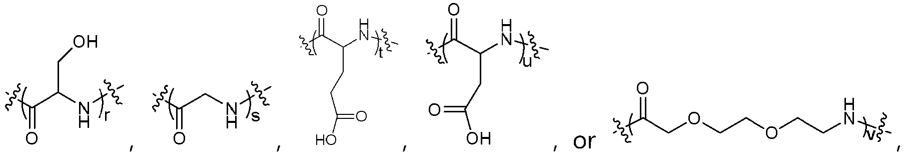 or any combination of two or more thereof;
wherein, when R^{1a} and R^{1b} are selected from hydrogen, and R^{1c} is selected from 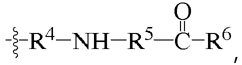 then R⁴ is selected from 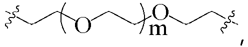 or R⁵ is selected from 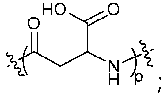
R⁶ is selected from a C₁₀₋₂₀ fatty acid;
m and n are integers, preferably integers from 0-10; p, q, r, s, t, u, v are selected from 1, 2, 3 or 4.

2. The GLP-1 compound according to claim 1, or an isomer thereof, or a racemate thereof, or a pharmaceutically acceptable salt thereof, **characterized in that**, when Z is selected from 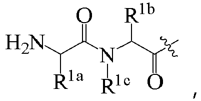 and said R^{1c} is selected from 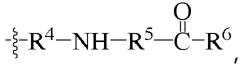 then the GLP-1 compound is selected from a compound shown in formula (II): 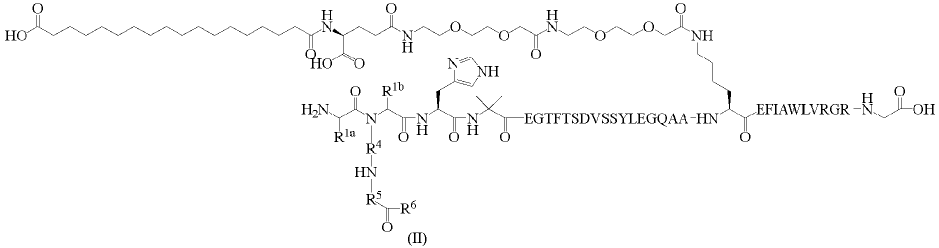
wherein, R^{1a} and R^{1b} are selected from hydrogen, or C₁₋₆ alkyl;
R⁴ is selected from C₁₋₆ alkyl, or 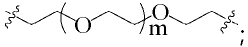
R⁵ is selected from 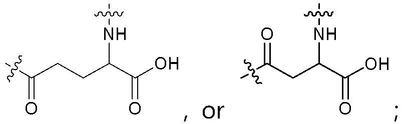 when R^{1a} and R^{1b} are selected from hydrogen, then R⁴ is selected from 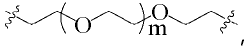 or R⁵ is selected from 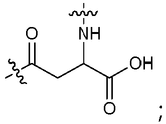
R⁶ is selected from a C₁₀₋₂₀ fatty acid;
m is selected from 0, 1, 2, 3, or 4.

3. The GLP-1 compound according to claim 1, or an isomer thereof, or a racemate thereof, or a pharmaceutically acceptable salt thereof, **characterized in that**, when Z is selected from 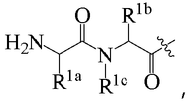 then the GLP-1 compound is selected from a compound shown in formula (III): 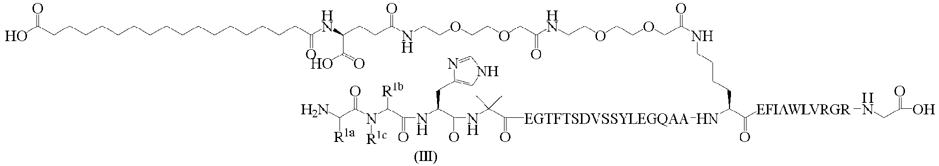
wherein, R^{1a} and R^{1b} are independently selected from hydrogen, C₁₋₆ alkyl, or 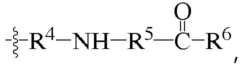 and said R^{1a} and R^{1b} are not simultaneously hydrogen or C₁₋₆ alkyl;
R^{1c} is selected from hydrogen, or C₁₋₆ alkyl;
R⁴ is selected from C₁₋₆ alkyl, or 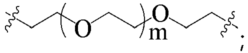
R⁵ is selected from one of 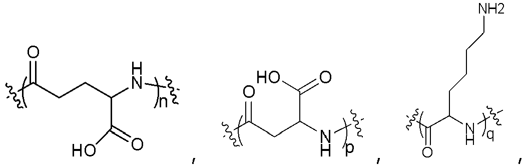 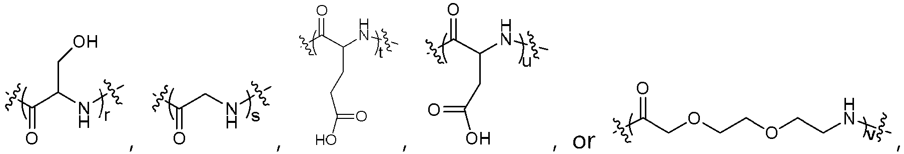 or any combination of two or more thereof;
R⁶ is selected from a C₁₀₋₂₀ fatty acid;
m is selected from 0, 1, 2, 3, or 4; n, p, q, r, s, t, u, v are selected from 1, 2, 3 or 4.

4. The GLP-1 compound according to claim 1, or an isomer thereof, or a racemate thereof, or a pharmaceutically acceptable salt thereof, **characterized in that**, when Z is selected from 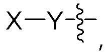 then the GLP-1 compound is selected from a compound shown in formula (VI): 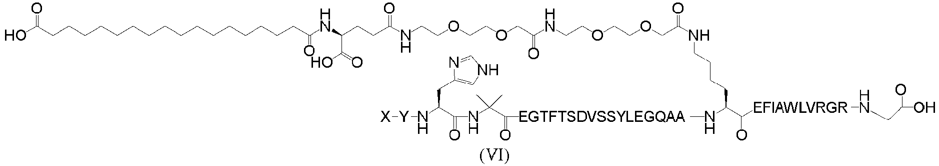
wherein, X and Y are independently selected from 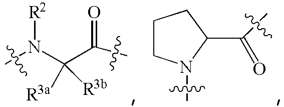 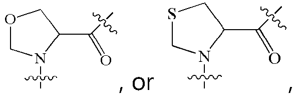 one of X and Y is selected from 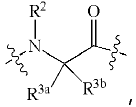 and a bond in the terminal group of X is linked to a hydrogen;
said R² is selected from hydrogen, C₁₋₆ alkyl, C₃₋₆ cycloalkyl, or 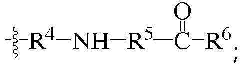
said R^{3a} **and** R^{3b} are each independently selected from hydrogen, C₁₋₆ alkyl, or 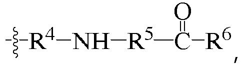 or alternatively, said R^{3a} and R^{3b} are cyclized, together with the atom to which they are attached, form a C₃₋₆ cycloalkyl; at least one of said R², R^{3a} and R^{3b} is selected from 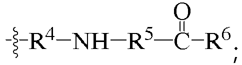
said R⁴ is selected from C₁₋₆ alkyl, or 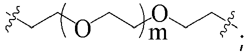
said R⁵ is selected from one of 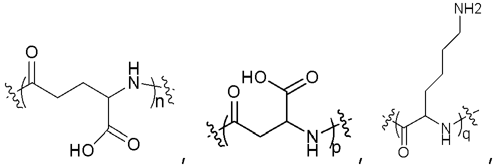 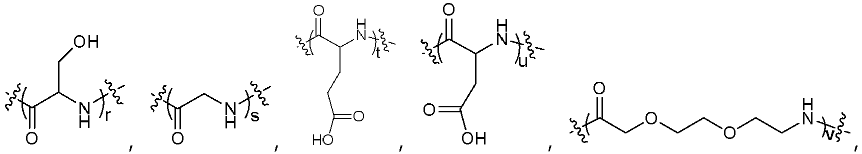 or any combination of two or more thereof;
R⁶ is selected from a C₁₀₋₂₀ fatty acid;
m is selected from 0, 1, 2, 3, or 4; n, p, q, r, s, t, u, v are selected from 1, 2, 3 or 4.

5. The GLP-1 compound according to any one of claims 1-4, or an isomer thereof, or a racemate thereof, or a pharmaceutically acceptable salt thereof, **characterized in that**, said C₁₋₆ alkyl is selected from methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neo-pentyl, n-hexyl, iso-hexyl, sec-hexyl, tert-hexyl, neo-hexyl, 2-methylpentyl, 1,2-dimethylbutyl, or 1-ethylbutyl.

6. The GLP-1 compound according to any one of claims 1-4, or an isomer thereof, or a racemate thereof, or a pharmaceutically acceptable salt thereof, **characterized in that**, said C₃₋₆ cycloalkyl is selected from cyclopropyl, cyclobutyl, cyclopentyl, or cyclohexyl.

7. The GLP-1 compound according to any one of claims 1-4, or an isomer thereof, or a racemate thereof, or a pharmaceutically acceptable salt thereof, **characterized in that**, m is 0 or 1; n is 1, p is 1;
said R^{1a}, R^{1b}, R^{1c}, R², R^{3a}, R^{3b} are each independently selected from hydrogen, methyl, or 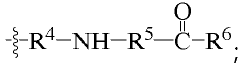
said R⁴ is selected from ethyl, butyl, or 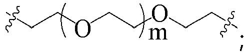
said R⁵ is selected from 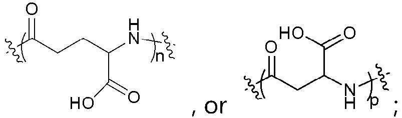 when R^{1a} and R^{1b} are selected from hydrogen, R^{1c} is selected from 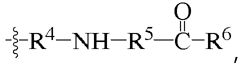 then R⁴ is selected from 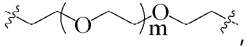 or R⁵ is selected from 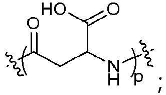
said R⁶ is selected from a C₁₅₋₂₀ fatty acid.

8. The GLP-1 compound according to claim 2, or an isomer thereof, or a racemate thereof, or a pharmaceutically acceptable salt thereof, **characterized in that**, m is 0 or 1;
when said R^{1a} and R^{1b} are selected from hydrogen, then said R⁴ is selected from 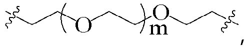 or R⁵ is selected from 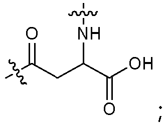
when said R^{1a} and R^{1b} are selected from methyl, then R⁴ is selected from ethyl, butyl, or 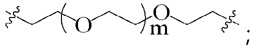 said R⁵ is selected from 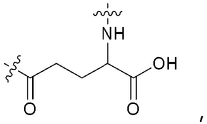 or 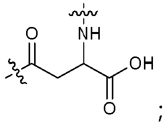
said R⁶ is selected from a C₁₅₋₂₀ fatty acid.

9. The GLP-1 compound according to claim 3, or an isomer thereof, or a racemate thereof, or a pharmaceutically acceptable salt thereof, **characterized in that**, m is selected from 0 or 1; n and p are selected from 1;
said R^{1a} and R^{1b} are independently selected from hydrogen, methyl, or 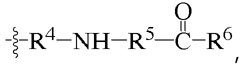 and said R^{1a} and R^{1b} are not simultaneously hydrogen or methyl;
said R^{1c} is selected from hydrogen or methyl;
said R⁴ is selected from ethyl, butyl, or 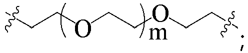
said R⁵ is selected from 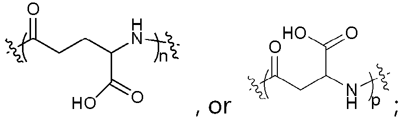
R⁶ is selected from a C₁₅₋₂₀ fatty acid.

10. The GLP-1 compound according to claim 4, or an isomer thereof, or a racemate thereof, or a pharmaceutically acceptable salt thereof, **characterized in that**, m is selected from 0 or 1; n and p are selected from 1;
said X and Y are independently selected from 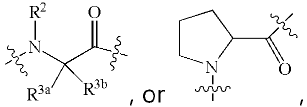 one of said X and Y is selected from 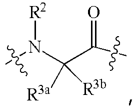 and a bond in the terminal group of X is linked to a hydrogen;
said R² is selected from hydrogen, methyl, or 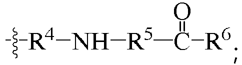
said R^{3a} and R^{3b} are each independently selected from hydrogen, methyl, or 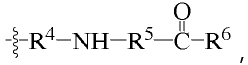 wherein, one of said R², R^{3a} and R^{3b} is selected from 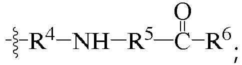
said R⁴ is selected from ethyl, butyl, or 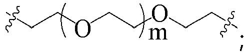
said R⁵ is selected from 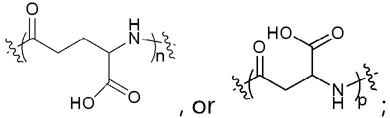
R⁶ is selected from a C₁₅₋₂₀ fatty acid.

11. The GLP-1 compound according to any one of claims 1-10, or an isomer thereof, or a racemate thereof, or a pharmaceutically acceptable salt thereof, **characterized in that**, said GLP-1 compound is selected from the compounds shown in Table 1.

12. A pharmaceutical composition, **characterized in that**, comprising a therapeutically effective amount of a compound according to any one of claims 1-11, or an isomer thereof, or a racemate thereof, or a pharmaceutically acceptable salt thereof, and a pharmaceutically acceptable carrier.

13. Use of a GLP-1 compound according to any one of claims 1-11, or an isomer thereof, or a racemate thereof, or a pharmaceutically acceptable salt thereof, in the manufacture of a medicine, specifically, in the manufacture of a medicine for treating a disease, wherein the disease is a chronic related disease selected from diabetes, obesity, non-alcoholic fatty liver disease and non-alcoholic steatohepatitis, cardiovascular disease, neurodegenerative disorders, chronic kidney disease, diabetic nephropathy, peripheral arterial disease, and/or heart failure.
